(19) Europäisches Patentamt
European Patent Office
Office européen des brevets

(11) Publication number: **0 264 911**
**A2**

(12) # EUROPEAN PATENT APPLICATION

(21) Application number: 87115347.4

(22) Date of filing: 20.10.87

(51) Int. Cl.[4]: **C07K 15/00**, C12P 21/00, C12N 5/00, C12N 15/00, G01N 33/574, G01N 33/577

The applicant has filed a statement in accordance with Rule 28 (4) EPC (issue of a sample only to an expert). Accession number(s) of the deposit(s): ATCC HB 9207; ATCC HB 9447; ATCC HB 9448; ATCC HB 9461; ATCC HB 9450; FERM BP 1522; FERM BP 1523.

The microorganism(s) has (have) been deposited with American Type Culture Collection and Fermentation Research Institute under number(s) ATCC HB 9207; ATCC HB 9447; ATCC HB 9448; ATCC HB 9461; ATCC HB 9450; FERM BP 1522; FERM BP 1523.

(30) Priority: **20.10.86 JP 250170/86**
**24.03.87 JP 71144/87**
**26.05.87 JP 130649/87**
**07.08.87 JP 198906/87**

(43) Date of publication of application:
**27.04.88 Bulletin 88/17**

(84) Designated Contracting States:
**CH DE ES FR GB IT LI NL SE**

(71) Applicant: **OTSUKA PHARMACEUTICAL CO., LTD.**
**9, Kandatsukasacho 2-chome**
**Chiyoda-ku Tokyo 101(JP)**

(72) Inventor: **Shin, Sadahito**
**40-20, Azanakazao Shinkirai Kitajima-cho**
**Itanogun Tokushima-ken(JP)**
Inventor: **Tachikawa, Tetsuya 13-6,**
**Azahigashinokaminomoto**
**Takabo Kitajima-cho**
**Itanogun Tokushima-ken(JP)**
Inventor: **Nakajima, Katsuyuki**
**3-23-9, Minami-cho**
**Maebashi-shi Gunma-ken(JP)**

(74) Representative: **Hansen, Bernd, Dr.rer.nat. et al**
**Hoffmann, Eitle & Partner Patentanwälte**
**Arabellastrasse 4**
**D-8000 München 81(DE)**

(54) **Anti-idiotype antibodies, process for preparing them and method of determining antibody.**

(57) This invention provides an anti-idiotype antibody to a specific antibody recognizing a carbohydrate linkage selected from the group consising of
the formula
NeuAcα2→3Galβ1→4GlcNAc(3→α1Fuc)β1→3Galβ1→4GlcNAc(3←α1Fuc)β1→3Galβ1→4Glc-,
the formula
Galβ1→4GlcNAc(3←α1Fuc)β1→3Galβ1→,
the formula
Fucα1→2Galβ1→4GlcNAc(3→α1Fuc)β1→3Galβ1→,
the formula
NeuAcα2→3Galβ1→3GlcNAc(4→α1Fuc)β1→3Galβ1→,
the formula
NeuAcα2→3Galβ1→3GlcNAc(4→α1Fuc)(6←α2NeuAc)β1→3Galβ1→ and
the formula
NeuAcα2→3Galβ1→3GlcNAc(6←α2NeuAc)β1→3Galβ1→,
a process for preparing the same and a method for determining a cancer antigen used the same.

EP 0 264 911 A2

# ANTI-IDIOTYPE ANTIBODIES, PROCESS FOR PREPARING THEM AND METHOD OF DETERMINING ANTI-BODY

The present invention relates to novel anti-idiotype antibodies which are useful for screening and diagnosing cancers.

With basic research conducted on carbohydrate linkage abnormalities due to the conversion of cells into cancers, attention has been directed in recent years to cancer antigens of carbohydrate nature as tumor markers. The carbohydrate linkages having the structures represented by the following formulae (1), (2), (3), (4), (5) and (6) are especially high in cancer specificity. Antigens having such carbohydrate linkages are very sharply reflected in the body fluid of cancer patients, while specific antibodies capable of recognizing the carbohydrate linkages have been established.

NeuAcα2→3 Galβ1→4 GlcNAc(3←α1 Fuc)β1→3 Galβ1→4 Glc
NAc(3←α1 Fuc)β1→3 Galβ1→4 Glc- (1)

Galβ1→4 GlcNAc(3←α1 Fuc)β1→3 Galβ1→ (2)

Fucα1→2 Galβ1→4 GlcNAc(3←α1 Fuc)β1→3 Galβ1→ (3)

NeuAcα2→3 Galβ1→3 GlcNAc(4←α1 Fuc)β1→3 Galβ1→ (4)

NeuAcα2→3 Galβ1→3 GlcNAc(4←α1 Fuc)(6←α2 NeuAc)β1→3
Galβ1→ (5)

NeuAcα2→3 Galβ1→3 GlcNAc(6←α2 NeuAc)β1→3 Galβ1→
(6)

Accordingly, it is expected that the detection of these carbohydrate linkages in biological fluids such as blood, urine, etc. are very useful for screening and diagnosing cancers. [Cancer Res., 46, 2619-2626 (1986); Biochem. Biophys. Res. Commun., 100, 1578-1586 (1981); J. Biol. Chem., 259, 4672-4680 (1984); J. Biol. Chem., 258, 11793-11797 (1983); Cancer Res., 43, 5489-5492 (1983); etc.].

As stated in the foregoing reports, antibodies hve already been established which are specific to the carbohydrate linkage of the formula (1) (hereinafter referred to as "Sialyl $Le^x$ -i"), to the carbohydrate linkage of the formula (2) (hereinafter referred to as "$Le^x$"), to the carbohydrate linkage of the formula (3) (hereinafter referred to as "Fucosyl $Le^x$"), to the carbohydrate linkage of the formula (4) (hereinafter referred to as "Sialyl $Le^a$"), to the carbohydrate linkage of the formula (5) (hereinafter referred to as "Disialyl $Le^a$"), or to the carbohydrate linkage of the formula (6) (hereinafter referred to as "Disialyl type-1"). Nevertheless, there is the problem that a purified antigen is not actually available which is usable as a labeled or insolubilized antigen or a standard antigen required for determining the carbohydrate linkage using the antibody. Although the glycolipids mentioned in the above reports are known as purified antigens having the Sialyl $Le^x$-i, $Le^x$, Fucosyl $Le^x$, Sialyl $Le^a$, Disialyl $Le^a$ or Disialyl type-1, the purification procedure for these glycolipids is very cumbersome and complex, which nevertheless gives a such a low yield as is available only for the determination of the structure. Thus, the products are in no way usable for the above determination of the carbohydrate linkage.

Accordingly, great limitations are involved in the technique for determining as a cancer antigen a tumor marker represented by Sialyl $Le^x$-i, $Le^x$, Fucosyl $Le^x$, Sialyl $Le^a$ , Disialyl $Le^a$ or Disialyl type-1, so that the determination technique still remains to be greatly improved in sensitivity and accuracy.

An object of the invention is to provide a novel anti-idiotype antibody useful for determining a cancer antigen.

Another object of the invention is to provide a method for determining a cancer antigen by an immunoassay with high sensitivity and high precision.

These and other objects of the invention will become apparent from the following description.

The present invention provides a novel anti-idiotype antibody responsive to a specific antibody which recognizes a carbohydrate linkage selected from the group consisting of Sialyl $Le^x$ -i, $Le^x$, Fucosyl $Le^x$, Sialyl $Le^a$ , Disialyl $Le^a$ and Disialyl type-1.

We have conducted intensive research to determine the above-mentioned cancer antigens with high sensitivity and high accuracy and found the following.

(1) The anti-idiotype antibody of the present invention responsive to a specific antibody recognizing the above-specified carbohydrate linkage as an antigen is highly specific to the autoantibody of the carbohydrate antigen and is therefore usable for determining the autoantibody very advantageously.

(2) Using the present anti-idiotype antibody of the type which dose-dependently inhibits the reaction between the carbohydrate antibody and the antibody thereto, the antigen and the antibody can be easily determined with high sensitivity and high accuracy since the anti-idiotype antibody can be advantageously used as a purified antigen.

(3) The present anti-idiotype antibody of the type which does not inhibit the reaction between the carbohydrate antigen and the antibody thereto is usable as a second antibody in determining the carbohydrate antigen using the antibody thereto. This assures the determination with greatly improved sensitivity.

(4) Accordingly, the carbohydrate antigen and the autoantibody thereto or cells producing the antibody can be determined with high sensitivity and high accuracy using such anti-idiotype antibody of the invention. Consequently, the cancer can be screened and diagnosed very advantageously.

(5) Further using the anti-idiotype antibody of the invention, the antibody which recognizes the above-specified carbohydrate linkage can be purified easily and specifically based on the binding specificity thereof.

We have conducted further research based on these findings and succeeded in establishing the anti-idiotype antibody of the invention.

The antibody is specified by its reaction specificity (antigen binding site to be recognized). The tertiary structure of such unique antigen binding site is formed in a hypervariable region in the immunoglobulin molecule. Antigenicity (antigenic structure) peculiar to the antibody is known (idiotype) which is determined by the structure of hypervariable region.

The anti-idiotype antibody is known as an antibody which is reactive with such idiotype. [Oudin, J. et al, Acad. Sci. (Paris), 257, 805 (1963); Jerne, N.K., An. Immunol., 125C, 373 (1974); Brown, C.A. et al., J. Immunol., 123, 2102 (1979); Jaffers, G. J. et al., Transplant Proc., 15, 646 (1983); Levy, R. et al., Fed. Proc. Fed. Am, Soc. Exp. Biol., 42, 2650 (1983); Koprowski, H. et al., Proc. Natl. Acad. Sci. USA 81, 216 (1984); etc.

The anti-idiotype antibody of the invention is specified as one which is reactive with or responsive to an antibody which is specified in that it recognizes a particular carbohydrate antigen and which acts as the antigen for the present anti-idiotype antibody. (The antibody which recognizes Sialyl $Le^x$-i will be hereinafter referred to as "AbI-(1)," the antibody which recognizes $Le^x$ as "AbI-(2)," the antibody which recognizes Fucosyl $Le^x$ as "AbI-(3)," the antibody which recognizes Sialyl $Le^a$as "AbI-(4)," the antibody which recognizes Disialyl $Le^a$as "AbI-(5)," and the antibody which recognizes Disialyl type-1 as "AbI-(6).") Accordingly, each of the anti-idiotype antibodies of the invention has specific reactivity with only one of the antibodies AbI-(1), AbI-(2), AbI-(3), AbI-(4), AbI-(5), AbI-(6).

The anti-idiotype antibodies of the invention includes one which recognizes a site away from the antigen binding site of the antibody AbI-(1), AbI-(2), AbI-(3), AbI-(4), AbI-(5), AbI-(6), ($\alpha$-type) and which therefore does not inhibit the reaction between the antigen (Sialyl $Le^x$-i, $Le^x$, Fucosyl $Le^x$, Sialyl $Le^a$, Disialyl $Le^a$ or Disialyl type-1) and the antibody AbI-(1), AbI-(2), AbI-(3), AbI-(4), AbI-(5) or AbI-(6). Further the anti-idiotype antibodies of the invention includes one which recognizes the antigen binding site of AbI-(1), AbI-(2), AbI-(3), AbI-(4), AbI-(5) or AbI-(6) or a site close thereto ($\gamma$-type), or one which is complementary to the antigen binding site and has the same structure as Sialyl $Le^x$-i, $Le^x$, Fucosyl $Le^x$, Sialyl $Le^a$, Disialyl $Le^a$ or Disialyl type-1 ($\beta$-type), with the result that the anti-idiotype antibody dose-dependently inhibits the reaction between various glycolipids as antigens and the antibody AbI-(1), AbI-(2), AbI-(3), AbI-(4), AbI-(5) or AbI-(6).

The carbohydrates, lipids, modes of combination thereof, etc. are herein expressed in terms according to IUPAC-IUB and generally used in the art or in terms which are conventional, as exemplified below.

NeuAc: N-acetylneuraminic acid
Gal: galactose
Glc: glucose
GlcNAc: N-acetylglucosamine
Fuc: fucose

The process for preparing the anti-idiotype antibodies of the invention will be described below in detail.

The anti-idiotype antibody of the invention can be prepared by a usual method of producing antibodies using Abl-(1), Abl-(2), Abl-(3), Abl-(4), Abl-(5) or Abl-(6) as an immunizing antigen. The antibody Abl-(1), Abl-(2), Abl-(3), Abl-(4), Abl-(5) or Abl-(6) for use as the immunizing antigen is not specifically limited insofar as it recognizes the corresponding structure of Sialyl $Le^x$-i, $Le^x$, Fucosyl $Le^x$, Sialyl $Le^a$ or Disialyl type-1, that is, it is enable to bind to the carbohydrate linkage, glycolipids or even glycoproteins containing the carbohydrate linkage. However, it is preferably a monoclonal antibody from the viewpoint of its specificity. Such antibodies are known in literature. For examples, the antibody Abl-(1) recognizing Sialyl Le $^x$-i is known as antibody "FH-6" disclosed in J. Biol. Chem., 259, 10511-10517 (1984). The antibody Abl-(2) recognizing Le $^x$ is known as antibody "FH-2" in J. Biol. Chem. 259 , 4681-4685 (1984). The antibody Abl-(3) recognizing Fucosyl $Le^x$ is known as antibody "AH-6" disclosed in J. Biol. Chem., 258 , 11793-11797 (1983). The antibody Abl-(4) recognizing Sialyl $Le^a$ is known as antibody "CA19-9" disclosed in Cancer Res, 43 , 5489-5492 (1983). The antibody recognizing Disialyl $Le^a$is known as antibody "FH-7" disclosed in J. Biol. Chem., 261, 5487-5495 (1986). The antibody recognizing Disialyl type-1 is known as antibody "FH-9" disclosed in Biochemistry, 25, 2859-2866 (1986). These antibodies can be prepared according to the these reports.

The antibody of the present invention is prepared preferably by immunizing a mammal with a specific antibody, such as antibody FH-6, FH-2, AN-6, CA19-9, FH-7 or FH-9, which recognizes a carbohydrate linkage herein specified, fusing the immune cells with plasmacytoma cells of a mammal to obtain hydridoma cells, cloning the cells, selecting a clone which produces the desired anti-idiotype antibody recognizing the above antibody, FH-6, FH-2, AH-6, Ca19-9, FH-7 or FH-9 and obtaining the desired anti-idiotype monoclonal antibody from the clone.

In the above process, the mammal to be immunized with an antigen, e.g. antibody FH-6, FH-2, AH-6, Ca19-9, FH-7 or FH-9, is not limited specifically. However, it is desirable to select a suitable animal in view of the amenability to the fusion of its cells with the plasmacytoma cell to be used. Generally, mice and rats are advantageously usable.

The mammal is immunized by a usual method, for example, by intravenously, subcutaneously or intraperitoneally giving the antibody, such as FH-6, to the mammal, more specifically by diluting the antibody, such as FH-6, with PBS or the like to a suitable concentration, causing the antibody to combine with a usual carrier, such as bovine serum albumin (BSA), when required and administering the antibody to the animal several times every 2 to 14 days at a total does of about 1 to 100 µg/animal. A usual adjuvant is usable for the administration. It is desirable that the spleen cells removed about 3 days after the final administration of the antigen can be used as immunized cells.

Plasmacytoma of mammals as the other host cells to be fused with the immune cells are various known cell lines including myeloma cells, such as $p^3$ ($p^3$/X63-Ag8) [Nature, 256, 495-497 (1975)], P3-U1 [Current Topics in Microbiology and Immunology, 81, 1-7 (1978)], NS-1 (Eur. J. Immunol., 6, 511-519 (1976)], MPC-11 [Cell, 8, 405-415 (1976)], SP2/0 [Nature, 276 , 269-270 (1978)], FO [J. Immunol. Meth., 35, 1-21 (1980)] X63. 6. 5.3. [J. Immunol., 123, 1548-1550 (1979)], S194 [J. Exp. Med., 148, 313-323 (1978)] etc., R210 [Nature, 277, 131-133 (1979)].

The fusion between the immune cells and plasmacyte cells is conducted basically by a known method, such as the method of Milstein et al (Method in Enzymology, Vol. 73, p3 (1981)). More specifically, the fusion reaction is conducted, for example, in a usual nutrient medium in the presence of a fusion promoting agent. Examples of useful fusion promoting agents are those usually used, such as polyethylene glycol (PEG) and hemagglutinating virus of Japan (HVJ). To achieve an improved fusion efficiency, auxiliary agents such as dimethyl sulfoxide can be added to the medium when so desired. The immunized cells and plasmacytoma cells are used in a usual ratio. For example, immunized cells are used in about 1 to about 10 times the amount of plasmacytoma cells. Examples of media useful for the fusion are RPMI-1640 medium and MEM medium which are used for proliferating plasmacytoma cells, and various other media which are used for incubating cells of this type. Generally it is desirable to use such media with the serum supplement, such as fetal calf serum (FCS), removed therefrom. To effect fusion, predetermined quantities of immunized cells and plasmacytoma cells are thoroughly mixed together in the medium, and a solution of PEG having an average molecular weight of about 1000 to about 6000 is admixed, as preheated to about 37°C, with the medium usually at a concentration of about 30 to about 60 W/V%. Subsequently, a suitable medium is admixed with the culture from time to time, followed by centrifuging and removal of the supernatant every time. Repetition of this procedure affords the desired hybridoma.

The desired hybridoma obtained is separated by incubation in a usual selection medium such as HAT medium (containing hypoxanthine, aminopterin and thymidine). The incubation with the HAT medium is conducted for a period of time, usually several days to several weeks, which is sufficient to extinguish the cells (e.g. unfused cells) other than the desired hybridoma cells. The hybridoma cells obtained are then subjected to the usual limiting dilution method to retrieve the clones producing the desired anti-idiotype antibody, followed by the production of monoclonal antibody.

The antibody-producing clones can be retrieved by various methods which are generally used for detecting antibodies, such as the ELISA method (Engvall, E., Meth. Enzymol., 70, 419-439 (1980), plaque method, spot mthod, agglutination reaction method, Ouchterlony method and radioimmunoassay (RIA).

The desired anti-idiotype antibody is identified as an antibody having specific reactivity with antibody FH-6, FH-2, AH-6, CA19-9, FH-7 or FH-9 but having no reactivity with any other antibody of the same type. For the detection of the desired antibody, it is advantageous to use antibody FH-6, FH-2, AH-6, CA19-9, FH-7 or FH-9 as purified.

When the anti-idiotype antibody of the invention already established by the present inventor is used, purified antibody FH-6 or the like can be obtained with ease by immunoprecipitation, affinity chromatography or like method. It is also advantageous to use as the immunizing antigen such purified antibody FH-6, FH-2, AH-6, CA19-9, FH-7 or FH-9.

In this way, the desired hybridomas producing anti-idiotype antibodies of the invention can be obtained, from which the hybridoma of particular type producing the desired anti-idiotype antibody can be selected when desired, based on the activity to inhibit the reaction between Sialyl $Le^x$-i and FH-6, between $Le^x$ and FH-2, between Fucosyl $Le^x$ and AH-6, between Sialyl $Le^a$ and CA19-9, between Disialyl $Le^a$ and FH-7 or between Disialyl type-1 and FH-9. These hybridomas can be subcultures in a conventional medium and are easily preservable for a prolonged period of time in liquid nitrogen.

The desired anti-idiotype antibody of the invention can be collected from the antibody-producing hybridoma as a culture supernatant by incubating the hybridoma in the usual manner, or as the ascites of a mammal which is amenable to the proliferation of the hybridoma, by administering the hybridoma. The former method is suitable for preparing the antibody with a high purity, while the latter method is suited to the quantity production of the antibody. When desired, the anti-idiotype antibody of the invention can be easily purified by a usual method such as salting out, absorption method, gel filtration, affinity chromatography or the like.

Using the anti-idiotype antibody of the invention, a carbohydrate antigen (i.e. Sialyl $Le^x$-i, $Le^x$, Fucosyl $Le^x$, Sialyl $Le^a$, Disialyl $Le^a$ or Disialyl type-1) and a specific antibody thereto, especially present in a body fluid, can be determined with very high sensitivity and high accuracy. Accordingly the anti-idiotype antibody provided by the invention is usable for screening and diagnosing cancers even at a very early stage.

Carbohydrate antigens or antibodies thereto can be determined using the anti-idiotype antibodies of the invention basically by usual immunological determination methods, for example, by contracting a sample such as a body fluid with the anti-idiotype antibody of the present invention and testing for reactivity. Such methods include, for example, RIA, enzyme immunoassay (EIA), agglutination method, flow cytometory (FCM), etc. These methods can be practiced by procedures and means which are generally employed, such as competition method, sandwich method, flow cytometer and the like.

Examples of body fluids useful as samples for the determination are blood, cell fluid, lymph, pleural fluid, peritoneal fluid, amniotic fluid, gastric juice, urine, pancreatic juice, spinal fluid, saliva, etc. Among these, blood, especially serum or blood plasma is desirable.

In the above immunological determination methods, the antigen or antibody in the determination system is insolubilized by being caused to chemically or physically react with an insoluble carrier in the usual manner. Examples of useful insoluble carriers are cellulose powder, Sephadex, Sepharose, polystylene, filter paper, carboxymethylcellulose, ion exchange resin, dextran, plastic film, plastic tube, nylon, glass beads, silk, polyamine-methyl vinyl ether-maleic acid copolymer, amino acid copolymer, ethylene-maleic acid copolymer and the like. The insolubilization is accomplished by the diazo method as a covalent method, peptide method (with use of an acid amino derivative, carboxy chloride resin, carbodiimide resin, maleic anhydride derivative, isocyanate derivative, cyan bromide-activated polysaccharide, cellulose carbonate derivative, condensation reagent or the like), alkylation method, carrier bonding method using a crosslinking reagent (using glutaraldehyde, hexamethylene isocyanate or the like as the crosslinking reagent), carrier binding method resorting to Ugl reaction and the like chemical reaction methods; ion bonding method using a carrier such as an ion exchange resin; and the physical adsorption method wherein glass beads or like porous glass is used as a carrier. When the agglutination method is used, the antibody of the invention is used as adsorbed by (sensitized with) particles, such as erythrocytes or latex particles, which are usually used for the agglutination test.

For labeling the antigen or antibody in the above determination methods, various common labeling agents are used which include radioactive substance, enzymatic labeling substances and fluorescent substances. Examples of useful labeling agents are radioactive substances such as $^{125}I$ or like radioactive iodine, fluorescent substances such as fluorescein isothiocyanate (FITC), tetramethylrhodamine isothiocyanate (TRITC), substituted-rhodamine isothiocyanate (XRITC), Rhodamine B isothiocyanate, dichlorotriazinefluorescein (DTAF) and the like, and enzymatic labeling substances such as peroxidase (POX), myeloperoxidase, chymotryspsinogen, procarboxypeptidase, glyceroaldehyde-3-phosphate dehydrogenase, amylase, phosphorylase, deoxyribonuclease (DNA-ase), ribonuclease (RNA-ase), alkali phosphatase, β-galactosidase and the like. Such labeling agents can be applied in the usual manner [see J. Biol. Chem., 254, 9349-9351 (1970); Nature, 194, 495 (1962); Acta. Endocrinol Suppl., 168, 206 (1972): Proc. Nat. Acad. Sci., USA, 58, 713 (1967); etc.]

The reaction of the determination methods can be conducted in the same manner as usual immunoreactions of this type. The solvent to be used in the determination system is a usual one which will not adversely affect the reaction. Examples of preferred solvents are citrate buffer, phosphate buffer, Tris-HCl buffer, acetate buffer and the like. The conditions for the immunoassay system are not limited specifically; those usually employed for determination methods of the present type are useful. More specifically, the immunoreaction is conducted generally at a temperature of up to 45°C, preferably at about 4 to about 40°C, for about 1 to about 40 hours. After the reaction, the combined product is separated from the free substances (B-F) by a known method. When the insolubilization method is resorted to, the solid phase can be separated from the liquid phase, for example, by centrifuging, filtration, washing or decantation. In other cases, the dextran-coated charcoal method, double antibody method or like conventional method is used.

The determination method of the invention wherein the anti-idiotype antibody is used has the following features.

(1) The anti-idiotype antibodies of the present invention include six kinds of antibodies, i.e. an anti-idiotype antibody which recognizes antibody FH-6 only, one which recognizes antibody FH-2 only, one which recognizes antibody AH-9 only, one which recognizes antibody CA19-9 only, one which recognizes antibody FH-7 only and one which recognizes antibody FH-9 only. Accordingly, for use in a system for determining the autoantibody to a carbohydrate antigen (i.e. Sialyl $Le^x$-i, $Le^x$, Fucosyl $Le^x$, Sialyl $Le^a$, Disialyl $Le^a$ or Disialyl type-1) present in the body fluid, the present anti-idiotype antibody is very advantageous as an antibody specific thereto. Usual systems for determining autoantibodies wherein anti-human IgG antibody or protein A is used involve many nonspecific binding reactions at all times and therefore have the drawback of low sensitivity and a large amount of sample required for determination. In contrast, the use of the present anti-idiotype antibody involves no nonspecific reaction, is therefore advantageous and provides a simple determination by a direct method, especially the convenient agglutination method. Furthermore, the present antibody as labeled with a fluorescent agent is applicable to the flow cytometory for the determination or fractionation of autoantibody producing cells.

(2) The antibodies of the invention of the type which dose-dependently inhibit the reaction between Sialyl $Le^x$-i and antibody FH-6 thereto, between $Le^x$ and antibody FH-2 thereoto, between Fucosyl $Le^x$ and antibody AH-6 thereto, between Sialyl $Le^a$ and antibody CA19-9 thereto, between Disialyl $Le^a$ and antibody FH-7 thereto and between Disialyl type-1 and antibody FH-9 thereto are satisfactorily usable as desired labeled or insolubilized antigen or standard antigen serving as purified antigens in systems for determining cancer antigens or antibodies thereto. The purified antigens thus provided for use in the assay systems assure greatly improved sensitivity and accuracy, further making it possible to employ the competition assay method with use of a labeled antigen, whereas this method was conventionally infeasible.

(3) The anti-idiotype antibodies of the invention of the type which produce no inhibitory effect on the reaction between Sialyl $Le^x$-i and antibody FH-6 thereto, between $Le^x$ and antibody FH-2 thereoto, between Fucosyl $Le^x$ and antibody AH-6 thereto, between Sialyl $Le^a$ and antibody CA19-9 thereto, between Disialyl $Le^a$ and antibody FH-7 thereto and between Disialyl type-1 and antibody FH-9 thereto are usable in systems wherein antibodies are used for determining cancer antigens, for the proliferation of antibodies, i.e. as second antibodies specific to antibodies, assuring greatly improved sensitivity for the determination.

Based on the foregoing features of the present anti-idiotype antibodies, suitable determination systems can be provided, for example, by the following methods.

Examples of systems for determining cancer antigens containing a carbohydrate linkage are given below. (1) An assay system wherein a labeled anti-idiotype antibody, a sample or standard substance, and an antibody serving as an antigen are reacted and thereafter subjected to double antibody method using an anti-rat antibody. (2) An assay system wherein an anti-idiotype antibody in the form of a solid phase, a sample or standard substance, and a labeled antibody serving as an immunogen (specific antibody) are reacted and thereafter used for the solid-phase competition method. (3) An assay system wherein an antigen coated bead, a sample or standard substance, and a labeled antibody serving as an immunogen are reacted, followed by the solid-phase competition method and then by proliferation using a labeled anti-idiotype antibody of the type which will not inhibit antigen-antibody reaction.

Examples of useful systems for determining autoantibodies to cancer antigens containing a carbohydrate linkage are as follows. (1) An assay system wherein an anti-idiotype antibody in the form of a solid phase and a sample or standard substance as reacted, followed by the sandwich assay method wherein the product is further reacted with a labeled anti-idiotype antibody. (2) An assay system wherein an anti-idiotype antibody in the form of a solid phase is reacted with a sample, followed by the sandwich method wherein the product is further reacted with a labeled anti-human IgG. (3) An assay system wherein a labeled antibody (immunogen), an anti-idiotype antibody, and a sample or standard antibody are reacted, followed by the liquid-phase competition method resorting to the double antibody method using an anti-rat IgG for B/F separation. (4) An assay system resorting to the competition method wherein an immobilized antibody (immunogen), a labeled anti-idiotype antibody, and a test sample or standard sample are reacted.

These determination methods can be practiced very conveniently using an assay kit incorporating the present anti-idiotype antibody. Cancers can be screened and diagnosed easily using this kit comprising a container containing the present anti-idiotype antibody. The antibody reagent of the kit may have incorporated therein a stabilizer, such as glycerol or bovine serum protein, and/or a preservative. Preferably, the antibody reagent is a lyophilized one. The kit may contain a solvent which is soluble in or miscible with water. A buffer for holding the reagent system at a predetermined pH after reconstitution and/or a preservative and/or a stabilizer for preventing the sample before use can be further incorporated in the antibody reagent. Although the buffer is not considered to be an essential component of the kit reagent, it is desirable to add such a substance to give a pH of about 4 to 8 to the system when the present method is practiced. While the reconstituting agent is preferably one containing water, the water can be partially or wholly replaced by a solvent which is miscible with water. Solvents which are miscible with water are well known to those skilled in the art. Examples of useful solvents are alcohols, glycols, glycol ethers, etc.

The present invention will be described in greater detail with reference to the following examples, reference examples and experimental examples.

## Example 1

Preparation of anti-idiotype antibody

(1) A phosphate-buffered NaCl solution (500 μl, pH 7.4) containing 100 μg of antibody FH-6 (Fukushi, Y., Nudelman, E., Levery, B.S. and Hakomori, S., J. Biol. Chem., 259, 10511-10517 (1984)) serving as antigen to an anti-idiotype antibody was admixed with the same amount of Freund complete adjuvant (DIFCO Laboratories, Detroit, Michigan, U.S.A.) to prepare a suspension. A quantity of the suspension corrresponding to 20 μg of FH-6 was subcutaneously administered to a Wistar rat. Another portion of the suspension corresponding to 20 μg of FH-6 was given to the rat two weeks thereafter. Further two weeks later, a solution of 200 μg of FH-6 in physiological saline was subcutaneously given. Three days after the last administration, the spleen was removed, and spleen cells were washed with RPMI-1640 medium three times.

Mouse myeloma cell strain P3U1 (Current Topics in Microbiology and Immunology, 81, 1-7, 1978) was similarly washed. A quantity of washed cells, i.e. 4 x $10^7$ cells, and 2 x $10^8$ spleen cells prepared above were mixed together in a 50-ml centrifugal tube and centrifuged at 200 G for 5 minutes. The supernatant was thereafter removed with a Pasteur pipette. A 2 ml quantity of RPMI-1640 solution containing 50 W/V% polyethylene glycol 1500 (Beringer Manheim Yamanouchi Co., Ltd.) and maintained at 37°C was added dropwise to the resulting sediment over a period of 1 minute. Subsequently, 1 ml of RPMI-1640 solution containing no FCS and maintained at 37°C was added to the mixture, followed by standing for 1 minute, then addition of 2 ml of the same solution and further standing for 2 minutes. With addition of 4 ml of the same solution, the mixture was allowed to stand for 4 minutes. To the mixture was then added 8 ml of RPMI-1640 containing 15% FCS, 0.05 g potency/liter of sulfuric acid streptomycin, 60000 U/liter of penicillin G potassium, 54 mg/liter of gentamicin and 1 mM pirvate (hereinafter referred to as "complete RPMI"), and the mixture was centrifuged at 200 G for 5 minutes. The sediment separated from the supernatant was suspended in complete RPMI maintained at 37°C at a concentration of 2 x $10^6$ spleen cells/ml. The suspension was placed on 24-well microplate (Coster Co., Ltd.), 0.1 ml in each well and incubated in 5% carbon dioxide incubator at 37°C. Twenty-four hours thereafter, 0.1 ml of complete RPMI medium containing 1.0 x $10^{-4}$M hypoxanthine, 4.0 x $10^{-7}$M aminopterin and 1.6 x $10^{-5}$M thymidine (hereinafter referred to as "HAT medium") was placed into each well. One-half of the supernatant was thereafter replaced by fresh HAT medium on the 3rd, 5th and 7th days. On the 9th day, one-half of the supernatant was replaced by complete RPMI medium containing 1.0 x $10^{-4}$M hypoxanthine and 1.6 x $10^{-5}$M thymidine (hereinafter referred to as "HT medium"). Similarly, one-half of the supernatant was replaced by HT medium on the 12th, 15th, 18th and 21st days and by complete RPMI medium on the 24th day. The culture was thereafter maintained for proliferation using complete RPMI medium.

The hybridomas thus obtained were cloned by the limiting dilution method. 2.5 x 10 hybridomas/ml and 4 x $10^8$ cells/ml of thymus of a mouse of Balb/c strain were suspended in complte RPMI medium, and the suspension was placed on a 96-well plate in an amount of 5 hybridomas/well and incubated. The hybridomas grown were further similarly cloned at a concentration of 0.5 hybridomas/well.

The clone producing the desired anti-idiotype antibody was retrieved by the following method. FH-6, antibody AH-6 (Abe, K., Mckibbin, J. M. and Hakomori, S., J. Biol., Chem., 258, 11793-11797 (1983)) and bovine serum albumin (BSA) were placed on 96-well plate as insolubilized. Into each well were placed 50 µl of the culture supernatant during cloning and 50 µl of phosphate buffer, followed by reaction with shaking at room temperature for 1 hour. The reaction mixture was thereafter washed with distilled water to remove the unreacted substances. Into each well was placed 100 µl of 6000-fold dilution of peroxidase-labeled anti-rat IgG (Cappel Co., Ltd.), followed by reaction with shaking at room temperature for 30 minutes. After removing the unreacted substances by washing with distilled water, 100 µl of 2.5 mg/ml solution of o-phenylenediamine in 0.01% $H_2O_2$ solution was placed into each well, followed by reaction at room temperature for 15 minutes while holding the plate at rest. The reaction was then terminated with 100 µl of 2N $H_2SO_4$. The microplate developing a color was measured at 492 nm using a microreader ("Titertek Multiskan R MCC," Flow Laboratories Inc., U.S.A.).

The clones producing the antibody of the present invention exhibiting a positive reaction (binding property) on FH-6 only were selected and subjected to the following inhibitory system screening. The supernatant (50 µl) of each clone was reacted with 200 µl (c.a. 50000 cpm) of $^{125}$I-labeled FH-6 at room temperature with shaking for 1 hour, one PC-9 extracts coated bead serving as an antigen was further reacted with shaking at room temperature for 1 hour, and the radioactivity of the bead was counted to check the activity of the clone to inhibit the binding between FH-6 and the antigen. With reference to the inhibitory activity of a system free from the culture supernatant as a control, clones were selected. The two screening systems and the limiting dilution method gave the desired hybridoma cells, i.e. two clones (clone Nos.: OAL-Ald-6A and OAL-Ald-6C) producing an antibody of the invention which did not inhibit the reaction between FH-6 and the antigen (Sialyl $Le^x$-i), and three clones (clone Nos.: OAL-Ald-6D, OAL-Ald-6E and OAL-Ald-6F). As a typical example, the hybridoma OAL-Ald-6F was deposited with American Type Culture Collection (ATCC symbol: HB 9207).

As representatives of the two types, OAL-Ald-6A and OAL-Ald-6F will be described below. The PC-9 extracts coated bead used as an antigen in the inhibitory system screening was prepared by extracting with perchloric acid (PCA) a culture supernatant of cancer cell strain PC-9 (Cancer Res., 42, 601-608 (1982)) known to express Sialyl $Le^x$-i, treating the extract with Sepharose CL-6B and causing a bead to adsorb (insolubilize) the resulting glycoprotein.

(2) Clone No.OAL-Ald-6A or OAL-Ald-6F obtained by the procedure (1) was incubated in complete RPMI medium with 5% carbon dioxide incubator at 37°C for 48 hours. The culture was centrifuged (3000 rpm., 10 minutes). In this way, culture supernatants containing the desired monoclonal antibodies (hereinafter referred to as "Ald-6A" and "Ald-6F", respectively from OAL-Ald-6A and -6F).

The antibodies were found to belong $IgG_2a$ class, as recognized by a rat monoclonal typing kit (product of Miles Scientific Co., Ltd.).

(3) Clone No.Ald-6A or Ald-6F (1 x $10^7$ cells) obtained by the procedure (1) was suspended in 0.5 ml of RPMI-1640 medium, and the suspension was intraperitoneally given to a nude mouse or nude rat. Two to three weeks later, the ascites was collected which contained Ald-6A or Ald-6F. The antibody concentration was about 0.2 to 5 mg/ml. The culture supernatanht obtained by the procedure (2) or the ascites obtained above and diluted two-fold was subjected to affinity chromatography using Sepharose bearing a solid phase of antibody FH-6 (prepared with use of BrCN-activated Sepharose CL-4B. product of Pharmacia). In this way, purified Ald-6A and Ald-6F were obtained.

Example 2

Preparation of anti-idiotype antibody

(1) A phosphate-buffered NaCl solution (500 µl, pH 7.5) containing 100 µl of antibody FH-2 (Biochem. Biophys. Res. Commun., 100, 1578-1586 (1981); J. Biol. Chem., 259 , 4672-4680 (1984)) serving as antigen to an anti-idiotype antibody was admixed with the same amount of Freund's complete adjuvant (DIFCO Laboratories, Detroit, Michigan, U.S.A.) to prepare a suspension. A quantity of the suspension corresponding to 20 µg of FH-2 was given subcutaneously to a Wistar rat. The rat was further immunized in the same manner as in Example 1, procedure (1), followed by cell fusion to obtain hybridomas, which were then cloned by the limiting dilution method.

The clone producing the desired anti-idiotype antibody was retrieved by the following method. Purified FH-2 and purified mouse IgM were placed on 96-well plate as insolubilized. Into each well were placed 50 µl of the culture supernatant during cloning and 50 µl of phosphate buffer, followed by reaction with shaking at room temperature for 1 hour. The clones producing the antibody of the invention exhibiting a positive reaction (binding property) on FH-2 only were thereafter selected by the same procedure as Example 1, procedure (1) and then subjected to the following inhibitory system screening. The supernatant (50 µl) of each clone was reacted with 200 µl (c.a. 50000 cpm) of $^{125}$I-labeled FH-2 with shaking at room temperature for 1 hour. A 200-µl portion of the reaction mixture was placed into a microplate having fixed thereto PC-7 cells serving as an antigen and reacted there with shaking at room temperature for 20 minutes. The activity to inhibit binding between FH-2 and the antigen was checked by counting the radioactivity on the cell-fixed microplate. With reference to the inhibitory activity of a system free from the culture supernatant as a control, clones were selected. The two screening systems and the limiting dilution method gave the desired hybridoma cells, i.e., one clone (clone No.: OAL-Ald-2X42) producing an anti-idiotype antibody of the invention which was low in activity to inhibit the reaction between FH-2 and antigen $Le^x$ , and four clones (clone Nos.: OAL-Ald-2X16, OAL-Ald-2X24, OAL-Ald-2X26 and OAL-Ald-2X50) producing an anti-idiotype antibody of the invention which was high in the above activity. As a typical example, the hybridoma OAL-Ald-2X26 was deposited in American Type Culture Collection (ATCC symbol: HB 9447). The microplate having fixed thereto PC-7 cells for use as an antigen in the inhibitory system screening was obtained by insolubilizing poly L-lysine and cells of cancer cell line PC-7 which is known as expressing the carbohydrate linkage structure $L^x$ (Yoshihiro Hayata et al., Igakuno Ayumi (Progress of Medicine), 90, 36, 1974; and Takayoshi Ohtani et al., Haigan (Lung Çancer), 16, 67, 1976), on the plate using glutaraldehyde.

(2) Each of the clones obtained by the above procedure (1), i.e., clone No.OAL-Ald-2X42, OAL-Ald-2X16, OAL-Ald-2X24, OAL-Ald-2X26 or OAL-Ald-2X50 was incubated in complete RPMI medium within 5% carbon dioxide gas incubator at 37°C for 48 hours. The resulting culture was centrifuged (3000 rpm, 10 minutes). In this way, culture supernatants were obtained which contained the respective desired monoclonal antibodies (hereinafter referred to as "Ald-1" "Ald-2", "Ald-3", "Ald-4" and "Ald-5").

OAL-Ald-2X16, OAL-Ald-2X24 and OAL-Ald-2X26 were found to belong to $IgG_1$ classe, and OAL-Ald-2X42 and OAL-Ald-2X50 to $IgG_2a$ class, as recognized by a rat monoclonal typing kit (product of Miles Scientific Chemical Co., Ltd.).

(3) Clone No.OAL-Ald-2X42, OAL-Ald-2X16, OAL-Ald-2X24, OAL-Ald-2X26 or OAL-Ald-2X50 (1 x $10^7$ cells) obtained by the procedure (1) was suspended in 0.5 ml of RPMI-1640 medium, and the suspension was intraperitoneally given to a nude mouse or nude rat. Two to three weeks later, the ascites was collected which contained Ald-1, Ald-2, Ald-3, Ald-4 or Ald-5. The antibody concentration was about 0.2 to 5 mg/ml. The culture supernatant obtained by the procedure (2), or the ascites obtained above and diluted two-fold was subjeced to affinity chromatography using Sepharose bearing a solid phase of antibody FH-2 (prepared with use of BrCN-activated Sepharose CL-4B, product of Pharmacia). In this way, purified Ald-1, Ald-2, Ald-3, Ald-4 and Ald-5 were obtained.

## Example 3

(1) A phosphate-buffered NaCl solution (500 µl, pH 7.4) containing 100 µl of antibody AH-6 (J. Biol. Chem., 258, 11793-11797, 1983) serving as antigen to an anti-idiotype antibody was admixed with the same amount of Freund's complete adjuvant to prepare a suspension. A quantity of the suspension corresponding to 20 µg of AH-6 was given subcutaneously to a Wistar rat. The rat was further immunized in the same manner as in Example 1, procedure (1) followed by cell fusion to obtain hybridomas, which were then cloned.

The clone producing the desired anti-idiotype antibody was retrieved by the following method. Purified AH-6 and purified mouse IgM were placed on 96-well plate as insolubilized. Into each well were placed 50 μl of the culture supernatant during cloning and 50 μl of phosphate buffer, followed by reaction with shaking at room temperature for 1 hour. The clones producing the antibody of the invention exhibiting a positive reaction on AH-6 only were thereafter selected by the same procedure as Example 1, procedure (1) and then subjected to the following inhibitory system screening. The supernatant (50 μl) of each clone was reacted with 200 μl (c.a. 50000 cpm) of $^{125}$I-labeled AH-6 with shaking at room temperature for 1 hour. One bead coated with a liver cancer tissue extracts serving as an antigen was added to the reaction mixture, followed by reaction with shaking at room temperature for 1 hour. The radioactivity of the bead was counted to check the clone for its activity to inhibit binding between AH-6 and the antigen. With reference to the inhibitory activity of a system free from the culture supernatant as a control, clones were selected. The two screening systems and the limiting dilution method gave the desired hybridoma cells, i.e., one clone (clone No.: OAL-Ald-4Y40) producing an anti-idiotype antibody for the invention which was low in activity to inhibit the reaction between AH-6 and antigen Fucosyl $LE^x$, and two clones (clone Nos.: OAL-Ald-4Y12 and OAL-Ald-4Y26) producing an anti-idiotype antibody of the invention which was high in the above activity. As a typical example, the hybridoma OAL-Ald-4Y12 was deposited in American Type Culture Collection (ATCC symbol: HB 9450).

The bead having a liver cancer cell extract coated thereon for use as an antigen and employed for the inhibitory system screening was prepared using liver tissue resulting from metastasis to the liver of colon cancer which was positive on reaction with AH-6 antibody, by subjecting the tissue to extraction with perchloric acid and causing a bead to adsorb (insolubilize) the resulting glycoprotein.

(2) Each of the clones obtained by the procedure (1), i.e., clone No.OAL-Ald-4Y40, OAL-Ald-4Y12 or OAL-Ald-4Y26 was incubated in complete RPMI medium within 5% carbon dioxide gas incubator at 37°C for 48 hours. The resulting culture was centrifuged (3000 rpm, 10 minutes). In this way, culture supernatants were obtained which contained the respective desired monoclonal antibodies (hereinafter referred to as "Ald-6," "Ald-7"" and "Ald-8").

OAL-Ald-4Y40 was found to belong to $IgG_1$ classe, and OAL-Ald-4Y12 and OAL-Ald-4Y26 to $IgG_{2}a$ class, as recognized by a rat monoclonal typing kit.

(3) Clone No.OAL-Ald-4Y40, OAL-Ald-4Y12 or OAL-Ald-4Y26 obtained by the procedure (1) was intraperitoneally given to a nude mouse or nude rat in the same manner as in the procedure (3) to obtain ascites containing Ald-6, Ald-7 or Ald-8. The fluid was then subjected to affinity chromatography using Sepharose bearing a solid phase of antibody AH-6. In this way, purified Ald-6, Ald-7 and Ald-8 were obtained.

Example 4

(1) A phosphate-buffered NaCl solution (500 μl, pH 7.4) containing 100 μl of antibody CA19-9 (Cancer Res., 43 , 5489-5492, 1983) serving as antigen to an anti-idiotype antibody was admixed with the same amount of Freund complete adjuvant to prepare a suspension. A quantity of the suspension corresponding to 20 μg of CA19-9 was given subcutaneously to a Wister rat. The rat was further immunized in the same manner as in Example 1, followed by cell fusion to obtain hybridomas, which were then cloned.

The clone producing the desired anti-idiotype antibody was retrieved by the following method. CA19-9 (J. B. C., 257 , 14365-14369, 1982; Cancer Research, 43, 5489-5492, 1983) and purified mouse IgM were insolubilized on 96-well plate. Into each well were placed 50 μl of the culture supernatant during cloning and 50 μl of phosphate buffer, followed by reaction with shaking at room temperature for 1 hour. The clones producing the antibody of the invention exhibiting a positive reaction on CA19-9 only were thereafter selected by the same procedure as Example 1, procedure (1) and then subjected to the following inhibitory system screening. The supernatant (50 μl) of each clone was reacted with 200 μl (c.a. 50000 cpm) of $^{125}$I-labeled CA19-9 with shaking at room temperature for 1 hour. One bead coated with a KATO-III cell extracts serving as an antigen was added to the reaction mixture, followed by reaction with shaking at room temperature for 1 hour. The radioactivity of the bead was counted to check the clone for its activity to inhibit binding between CA19-9 and the antigen. With reference to the inhibitory activity of a system free from the culture supernatant as a control, clones were selected. The two screening systems and the limiting dilution method gave the desired hybridoma cells, i.e., a clone (clone No.: OAL-Ald-19A4) producing an anti-idiotype antibody of the invention of the type inhibiting the reaction between CA19-9 and antigen (Sialyl $Le^a$) and two clones (clone Nos.: OAL-Ald-19A7 and OAL-Ald-19A10) producing an anti-idiotype antibody of the invention inhibiting the reaction. As a typical example, the hybridomas OAL-Ald-19A4 and OAL-Ald-19A10 were deposited in American Type Culture Collection (ATCC symbol: HB 9461 and HB 9448).

KATO-III used as an antigen for the inhibiting system screening was prepared from cultured cells of cancer cell line KATO-III which is known as expressing Sialyl $Le^a$ (Morimasa Sekiguchi et al., Soshiki Baiyo (Tissue Culture), 9(6), 201-207, 1983), by subjecting the cells to extraction with PBS and to treatment with PNA-Sepharose CL-6B and causing a bead to adsorb (insolubilize) the resulting glycoprotein.

(2) Clone No.OAL-Ald-19A4, OAL-Ald-19A7 or OAL-Ald-19A10 obtained by the procedure (7) was incubated in complete RPMI medium within 5% carbon dioxide gas incubator at 37°C for 48 hours. The resulting culture was centrifuged (3000 rpm, 10 minutes). In this way, culture supernatants were obtained which contained the respective desired monoclonal antibodies (hereinafter referred to as "Ald-9," "Ald-10" and "Ald-11").

OAL-Ald-19A4 and OAL-Ald-19A7 were found to belong to $IgG_{2}a$ classe, and OAL-Ald-19A10 to $IgG_1$ class, as recognized by a rat monoclonal typing kit.

(3) Clone No.OAL-Ald-19A4, OAL-Ald-19A7 or OAL-Ald-19A10 ($1 \times 10^7$ cells) obtained by the procedure (7) was intraperitoneally given to a nude mouse or nude rat in the same manner as in the procedure (3) to obtain ascites containing Ald-9, Aid-10 or Ald-11. The fluid was then subjected to affinity chromatography using Sepharose bearing a solid phase of antibody CA19-9. In this way, purified Ald-9, Ald-10 and Ald-11 were obtained.

Example 5

(1) A phosphate-buffered NaCl-solution (500 µl, pH 7.4) containing 100 µl of antibody FH-7 (J. Biol. Chem., 261, 5487-5495, 1986) serving as an antigen to an anti-idiotype antibody was admixed with the same amount of Freund's complete adjuvant to prepare a suspension. A quantity of the suspension corresponding to 20 µg of FH-7 was given subcutaneously to a Wistar rat. The rat was further immunized in the same manner as in Example 1-(1), followed by cell fusion to obtain hybridomas, which wre then cloned.

The clone producing the desired anti-idiotype antibody was retrieved by the following method. Purified FH-7 antibody and purified mouse IgG were insolubilized on 96-well plate. Into each well were placed 50 µl of the culture supernatant during cloning and 50 µl of phosphate buffer, followed by reaction with shaking at room temperature for 1 hour. The clones producing the antibody of the invention exhibiting a positive reaction on FH-7 only were thereafter selected by the same procedure as Example 1, procedure (1) and then subjected to the following inhibitory system screening. The supernatant (50 µl) of each clone was reacted with 200 µl (c.a. 100000 cpm) of $^{125}I$-labeled FH-7 with shaking at room temperature for 1 hour. One bead coated with meconium extrct serving as an antigen was added to the reaction mixture, followed by reaction with shaking at room temperature for 1 hour. The radioactivity of the bead was counted to check the clone for its activity to inhibit binding between FH-7 and the antigen. With reference to the inhibitory activity of a system free from the culture supernatant as a control, clones were selected. The two screening systems and the limiting dilution method gave the desired hybridoma cells, i.e., one clone (clone Nos.: OAL-Ald-7A1 and OAL-Ald-7A2) producing an anti-idiotype antibody of the invention of the type inhibiting the reaction between FH-7 and antigen (Sialyl $Le^a$) and a clone (clone No.: OAL-Ald-7A3) producing an anti-idiotype antibody of the invention not inhibiting the reaction. As a typical example, the hybridoma OAL-Ald-7A1 was deposited in fermentation Research Institute, Japan, with deposition number FERM BP-1522.

The bead used as an antigen in the inhibitory system screen and coated with meconium extract was prepared by adding 5 g of 0.6 M perchloric acid to 1 g of meconium which was positive on reaction with FH-7, homogenizing the mixture by a Warning blender, centrifuging the mixture at 150000 x g for 30 minutes, followed by neutralization of the resulting supernatant, dialysis and lyophilization. The product was further subjected to gel filtration using Sepharose CL-6B, a void fraction was collected and a bead was caused to adsorb the glycoprotein obtained.

(2) Clone No.OAL-Ald-7A1, OAL-Ald-7A2 or OAL-Ald-7A3 obtained above was incubated in complete RPMI medium within 5% carbon dioxide gas incubator at 37°C for 48 hours. The resulting culture was centrifuged (3000 rpm, 10 minutes). In this way, culture supernatants were obtained which contained the respective desired monoclonal antibodies (hereinafter referred to as "AlD-7A1," "Ald-72" and "Ald-7A3").

Each of the antibody was found to belong to $IgG_{2}a$ classe as recognized by a rat monoclonal typing kit (product of Miles Scientific Chemical Co., Ltd.).

(3) Clone No.OAL-Ald-7A1, OAL-Ald-7A2 or OAL-Ald-7A3 ($1 \times 10^7$ cells) obtained in Example 5-(1) was suspended in 0,5 ml of RPMI-1640 medium, and the suspension was intraperitoneally given to a nude mouse or nude rat. Two to three weeks later, the ascites was collected which contained Ald-7A1, Ald-7A2 or Ald-7A3. The antibody concentration was about 0.2 to about 5.0 mg/ml. The culture supernatant obtained by the procedure (2) or the ascites obtained above and diluted two-fold was subjected to affinity chromatography using Sepharose bearing a solid phase of antibody FH-6 (prepared with use of BrCN-activated Sepharose CL-4B, product of Pharmacia). In this way, purified Ald-7A1, Ald-7A3 and Ald-7A3 were obtained.

Example 6

Preparation of anti-idiotype antibody

(1) A phosphate-buffered NaCl solution (500 µl, pH 7.4) containing 100 µl of antibody FH-9 (Biochemistry, 25, 2859-2866, 1986) serving as antigen to an anti-idiotype antibody was admixed with the same amount of Freund's complete adjuvant to prepare a suspension. A quantity of the suspension corresponding to 20 µg of FH-9 was given subcutaneously to a Wistar rat. The rat was further immunized in the same manner as in Example 1-(1), followed by cell fusion to obtain hybridomas, which were then cloned.

The clone producing the desired anti-idiotype antibody was retrieved by the following method. Purified FH-9 and purified mouse $IgG_1$ were placed on 96-well plate as insolubilized. Into each well were placed 50 µl of the culture supernatant during cloning and 50 µl of phosphate buffer, followed by reaction with shaking at room temperature for 1 hour. The clones producing the antibody of the invention exhibiting a positive reaction on FH-9 only were thereafter selected by the same procedure as Example 1, procedure (1) and then subjected to the following inhibitory system screening. The supernatant (50 µl) of each clone was reacted with 200 µl (c.a. 50000 cpm) of $^{125}I$-labeled FH-9 with shaking at room temperature for 1 hour. A microplate having fixed thereto PC-7 cells serving as an antigen and reacted there with shaking at room temperature for 1 hour. The radioactivity on the cell fixed microplate was counted to check the clone for its activity to inhibit binding between FH-9 and the antigen. With reference to the inhibitory activity of a system free from the culture supernatant as a control, clones were selected. The two screening systems and the limiting dilution method gave the desired hybridoma cells, i.e., one clone (clone No.: OAL-Ald-9A1) producing an anti-idiotype antibody of the invention of the type inhibiting the reaction between FH-9 and antigen Disialyl type-1). The hybridoma OAL-Ald-9A1 was deposited in Fermentation Research Institute, Japan, with deposition number FERM BP-1523.

PC-7 used in the inhibitory system screening as an antigen was prepared from culture cells of lung cancer cell line PC-7 by placing the cells on a microplate bearing a solid phase of polylysine in a quantity of $10^5$ cells/well and fixing the cells with gluraraldehyde.

(2) Clone No.OAL-Ald-9A1 obtained by the procedure (1) was incubated in complete RPMI medium within 5% carbon dioxide gas incubator at 37°C for 48 hours. The resulting culture was centrifuged (3000 rpm, 10 minutes). In this way, a culture supernatant was obtained which contained the desired monoclonal antibody (hereinafter referred to as "Ald-9A1").

OAL-Ald-9A1 was found to belong to $IgG_{2}a$ class as recognized by a rat monoclonal typing kit.

(3) Clone No.OAL-Ald-9A1 obtained by the procedure (1) was intraperitoneally given to a nude mouse or nude rat in the same manner as in Example 1-(3). Two to three weeks later, the ascites was collected which contained Ald-9A1. The antibody concentration was about 0.2 to 5 mg/ml. The culture supernatant obtained by the procedure (2) or the ascites obtained above and diluted two-fold was subjected to affinity chromatography using Sepharose bearing a solid phase of antibody FH-9 (prepared using BrCN-activated Sepharose C1-4B, product of Pharmacia), whereby purified Ald-9A1 was obtained.

Reference Example 1

(1) Preparation of Insolubilized antibody

Purified antibody Ald-6A or Ald-6F obtained in Example 1 was adjusted to a concentration of 5 µg/protein/ml with 50 mM PBS (pH 7.4) containing 0.15M NaCl and 0.05% $NaN_3$.

Ten thousand polystyrene beads (4 mm in diameter, product of Sekisui Chemical Co., Ltd.) were thoroughly washed with 1/1000N NaOH solution containing 30% ethanol and further with distilled water. The beads were then washed thoroughly with 1/1000N NaCl solution and thereafter with distilled water.

Eight hundred beads were added to 100 ml of the antibody solution, and the mixture was stirred for 2 hours and then allowed to stand at 4°C overnight. The beads were filtered off, washed with physiological saline and placed into 50 mM PBS (pH 7.4) containing 0.5% crystalline BSA (Seikagaku Kogyo Co., Ltd.). The mixture was stirred for 2 hours and thereafter allowed to stand at 4°C overnight. The beads were filtered off and thoroughly washed to obtain an insolubilized antibody.

(2) Preparation of $^{125}$I-labeled antibody

One mCi of Na$^{125}$I (NEN Co., Ltd.) was added to a solution of 100 µg of purified antibody Ald-6A or Ald-6F in 0.1M borate buffer (pH 8.2). A dichloromethane solution (40 µl) of 1 ml/ml of Iodogen (Pierce Co., Ltd.) was placed into a glass test tube, which was then dried by removing the solvent in a nitrogen gas stream. The antibody solution was placed into the test tube and then allowed to stand with ice cooling for 5 minutes for reaction. The reaction product was placed into another test tube to terminate the reaction and then subjected to gel filtration (Sepharose CL-6B, eluent: 50 mM phosphate buffer containing 0.15M NaCl, 0.1% BSA and 0.02% $NaN_3$), whereby IgG fractions matching in radioactivity peak were collected to obtain $^{125}$I-labeled antibody, which was tested for reactivity using antibody FH-6.

Satisfactory samples of $^{125}$I-labeled antibodies were also prepared by the chloramine T method (Nature, 194, 496,1962) and the Bolton-Hunter method (Biochem. J., 89, 114,1963).

(3) Preparation of antibody labeled with enzyme

Purified antibody Ald-6A or Ald-6F (10 mg) was dissolved in 1 ml of 1M phosphate buffer (pH 6.8), and the solution was gently stirred with addition of the same buffer containing 10 mg/ml of peroxidase. After adding 50 µl of 1% glutaraldehyde solution dropwise to the mixture, the mixture was incubated ato room temperature for 2 hours. The reaction mixture was dialyzed against saline at 4°C for one day to obtain a peroxidase-labeled antibody.

Satisfactory samples of peroxidase-labeled antibody were also prepared by the periodic acid crosslinking method (J. Histochem. Cytochem., 22,1084-1091,1974), the maleimide crosslinking method (J. Histochem. Cytochem., 78, 235-237, 1975), the isocyanate crosslinking metho (J. Histochem. Cytochem., 21, 233-240, 1973) and the benzoquinone crosslinking method (Ann. Immunol., 127C, 197-208, 1976). It was also possible to label the enzyme with other enzymes such as β•Ggalactosidase, alkali phosphatase, maleate dehydrogenase, glucose-6-phosphate dehydrogenase, glucose oxidase, acetylcholine esterase, glucoamylase and lysozyme.

(4) Preparation of fluorescence-labeled antibody

Ten mg of purified anti-idiotype antibody Ald-6F prepared in Example 1-(2) was dissolved in 1 ml of 0.05M carbonate buffer (pH 9.5). A solution prepared by dissolving FITC (fluorescein isothiocyanate) in the same buffer at a concentration of 100 µg/ml was added dropwise to the solution, and the mixture was stirred at room temperature for 1 hour. The mixture was thereafter dialyzed for 1 day using 0.005M phosphate buffer and then purified by ion exchange using DEAE-Sepharose to obtain an FITC-labeled anti-idiotype antibody.

The antibody was also satisfactorily labeled with RITC (tetramethylrhodamine isothiocyante) by the same method as above.

Reference Example 2

(1) Preparation of insolubilized antibody

Purified anti-idiotype antibody AId-1, AId-2, AId-3, AId-4 or AId-5 prepared in Example 2-(3) was dissolved to a concentration of 5 µg protein/ml in 50 mM PBS (pH 7.4) containing 0.15M NaCl and 0.05% $NaN_3$. The same procedure as Reference Example 1-(1) was thereafter repeated to obtain an insolubilized antibody.

(2) Preparation of $^{125}I$-labeled antibody

Purified antibody AId-1, AId-2, AId-3, AId-4 or AId-5 (100 g) obtained in Example 2-(3) was dissolved in 0.1 ml of 0.1M borate buffer (pH 8.2). One mCi of $Na^{125}I$ (NEN Co., Ltd.) was added to the solution. The same procedure as Reference Example 1-(2) was then repeated to prepare a $^{125}I$-labeled antibody.

(3) Preparation of enzyme-labeled antibody

Ten ml of purified anti-idiotype antibody AId-1, AId-2, AId-3, AId-4 or AId-5 obtained in Exmaple 2-(3) was dissolved in 1 ml of 0.1M phosphate buffer (pH 6.8), and a solution of 10 mg/ml of peroxidase in the same buffer was added to the solution. The same procedure as Reference Example 1-(3) was thereafter repeated to obtain a peroxidase-labeled antibody.

(4) Preparation of fluorescence-labeled antibody

Ten mg of purified anti-idiotype antibody AId-1, AId-2, AId-3, AId-4 or AId-5 obtained in Example 2-(3) was dissolved in 1 ml of 0.05M carbonate buffer (pH 9.5). A solution prepared by disssolving FITC fluorescein isothiocyanate) in the same buffer at a concentration of 100 µg/ml was added dropwise to the solution, and the mixture was stirred at room temperature for 1 hour. The same procedure as Reference Example 1-(4) was thereafter repeated to obtain an FITC-labeled anti-idiotype antibody.

Reference Example 3

(1.) Preparation of insolubilizing antibody

Antibody AId-6, AId-7 or AId-8 obtained in Example 3-(3) was treated in the same manner as in Reference Example 1-(1) to prepare as insolubilized antibody.

(2) Preparation of $^{125}I$-labeled antibody

Antibody AId-6, AId-7 or AId-8 obtained in Example 3-(3) was treated in the same manner as in Refernce Example 1-(2) to prepare a $^{125}I$-labeled antibody.

(3) Preparation of enzyme-labeled antibody

Purified anti-idiotype antibody AId-6, AId-7 or AId-8 obtained in Example 3-(3) was treated in the same manner as in Example 1-(3) to prepare a peroxidase-labeled antibody.

(4) Preparation of fluorescence-labeled antibody

Purified anti-idiotype antibody AId-6, AId-7 or Aid-8 obtained in Example 3-(3) was also treated in the same manner as in Refernce Example 1-(4) to prepare a satisfactory FITC-labeled anti-idiotype antibody.

Reference Example 4

(1) Preparation of insolubilized antibody

Antibody Ald-9, Ald-10 or Aid-11 obtained in Example 4-(3) was treated in the same manner as in Reference Example 1-(1) to prepare an insolubilized antibody.

(2) Preparation of $^{125}I$-labeled antibody

Antibody Ald-9, Ald-10 or Aid-11 obtained in Example 4-(3) was treated in the same manner as in Reference Example 1-(2) to prepare a $^{125}I$-labeled antibody.

(3) Preparation of enzyme-labeled antibody

Purified anti-idiotype antibody Ald-9, Ald-10 or Ald-11 obtained in Example 4-(3) was treated in the same manner as in Reference Example 1-(3) to prepare a peroxidase-labeled antibody.

(4) Preparation of fluorescence-labeled antibody

Purified anti-idiotype antibody Ald-9, Ald-10 or Ald-11 obtained in Example 4-(3) was also treated in the same manner as in Reference Example 1-(4) to prepare a satisfactory FITC-labeled anti-idiotype antibody.

Reference Example 5

(1) Preparation of insolubilized antibody

Antibody Ald-7A1, Ald-7A2 or Ald-7A3 obtained in Example 5-(3) was treated in the same manner as in Reference Example 1-(1) to prepare an insolubilized antibody.

(2) Preparation of $^{125}I$-labeled antibody

Antibody Ald-7A1, Ald-7A2 or Ald-7A3 obtained in Example 5-(3) was treated in the same manner as in Reference Example 1-(2) to prepare a $^{125}I$-labeled antibody.

(3) Preparation of enzyme-labeled antibody

Purified anti-idiotype antibody Ald-7A1, Ald-7A2 or Ald-7A3 obtained in Example 5-(3) was treated in the same manner as in Reference Example 1-(3) to prepare a peroxidase-labeled antibody.

(4) Preparation of fluorescence-labeled antibody

Purified anti-idiotype antibody Ald-7A1, Ald-7A2 or Ald-7A3 obtained in Example 5-(3) was also treated in the same manner as in Reference Example 1-(4) to prepare a satisfactory FITC-labeled anti-idiotype antibody.

Reference Example 6

(1) Preparation of insolubilized antibody

Antibody AId-9A1 obtained in Example 6-(3) was treated in the same manner as in Reference Example 1-(1) to prepare an insolubilized antibody.

(2) Preparation of $^{125}I$-labeled antibody

Antibody AId-9A1 obtained in Example 6-(3) was treated in the same manner as in Reference example 1-(2) to prepare a $^{125}I$-labeled antibody.

(3) Preparation of enzyme-labeled antibody

Purified anti-idiotype antibody AId-9A1 obtained in Example 6-(3) was treated in the same manner as in Reference Example 1-(3) to prepare a peroxidase-labeled antibody.

(4) Preparation of fluorescence-labeled antibody

Purified anti-idiotype antibody AId-9A1 obtained in Example 6-(3) was also treated in the same manner as in Reference Example 1-(4) to prepare a satisfactory FITC-labeled anti-idiotype antibody.

Test Example 1

Dilutions (X100, X300, X900, X270, X8100 and X24300) of AId-6A or AId-6F culture supernatant were preparing using complete RPMI-1640 medium. Tubes each containing 50 µl of each dilution and 200 µl of phosphate buffer (pH 7.4) were prepared. Immobilized beads of FH-6 antibody (J. B. C., 258, 11793, 1983),
FH-2 antibody (B. B. R. C., 109, 36, 1982),
FH-4 antibody (J. B. C., 259, 4681, 1984),
1A-4 antibody (Science, 220, 509, 1983),
CA19-9 antibody (J. B. C., 257, 14865, 1982),
CA125 antibody (Cancer Res., 44, 2813, 1984),
Ca15-3 antibody (Prot. Biol. Fluids, 13, 1013, 1984),
BALB/C mouse IgM antibody and BSA
were placed into the respective tubes. Each of the tubes thus prepared was shaken at room temperature for 2 hours for reaction.

Subsequently, the contents of the tube were washed with 3 ml of distilled water two times to remove unreacted AId-6A or AId-6F, and 20 µl of peroxidase-labeled anti-rat IgG (product of Cappel, X18000 dilution) was placed into the tube, which was then shaken at room temperature for 1 hour for reaction.

Unreacted peroxidase-labelled anti-rat IgG was removed by washing with 3 ml of distilled water two times. The immobilized bead was then placed into another tube. A solution (500 µl) prepared by dissolving o-phenylenediamine in 0.01% $H_2O_2$ solution to a concentration of 2.5 mg/ml was placed into the tube. After allowing the mixture to stand at room temperature for 30 minutes, 2.0 ml of 1.3N $H_2SO_4$ was placed into the tube to terminate the reaction. The reaction mixture was then checked for absorbance at 492 nm.

Consequently, AId-6A and AId-6F reacted with the immobilized bead of FH-6f antibody only as seen in Fig. 1, failing to react with any of the other beads of antibodies and BSA. This indicates that AID-6A and AId-6F are anti-idiotype antibodies having specific reactivity with FH-6.

Test Example 2

FH-6 (X10 dilution of hybridoma culture supernatant), and OAL-Ald-6A or OAL-Ald-6F (each of X2, X6, X18, X58, X162, X486 and X1458 dilutions) were mixed together in equal amounts and reacted at room temperature for 15 minutes. An immobilized bead having supported thereon 5 ng of a purified glycolipid (6B ganglioside) having Sialyl $Le^x$-i, 15 ng of cholesterol and 25 ng of phosphatidyl choline was added to the reaction mixture. After washing the bead, FH-6 remaining on the bead on reaction was quantitatively determined using FITC-labeled mouse IgG + M and Pandex device.

Fig. 2 shows the result achieved by Ald-6F. The result indicates that Ald-6F is an antibody of the type which dose-dependently inhibit binding between Sialyl $Le^x$ -i and FH-6. The above test revealed that Ald-6A totally failed to inhibit the above binding.

Test Example 3

(1) System for determining antibody recognized Sialyl $Le^x$-i using present antibody as specific antibody

(a) A 50-µl quantity of each of standard solutions of FH-6 in goat serum, having varying concentrations, was reacted with an immobilized bead of Ald-6F in 50 mM PBS (pH 7.4) buffer containing 0.1% BSA and 0.05% $NaN_3$ with shaking at room temperature for 2 hours. After removing the unreacted substances by washing, 200 µl of a solution of $^{125}I$-labeled antibody (Ald-6F) obtained above (about 100,000 cpm) in the same buffer was reacted with the bead with shaking at room temperature for 2 hours. After removing the unreacted substances by washing, the radioactivity of the bead was measured by a gamma counter. Fig. 3 shows a standard curve prepared from the result achieved.

(b) To 50 µl of FH-6 antibody standard prepared in the same manner as in (a) was added 200 µl of a solution of $^{125}I$-labeled Ald-6F in the same buffer as used in (a) (about 20000 cpm), and the mixture was reacted with shanking at room temperature for 2 hours. An immobilized bead of FH-6 was further added to the reaction mixture, followed by reaction in the same manner as above for 1 hour. The bead was then washed and thereafter checked for radioactivity by a gamma counter.

Fig. 4 shows a standard curve prepared from the result. In the diagram, Bo represents 0 µ/ml of standard, meaning the binding cpm when a blank solution is used. In comparison with Bo, B represents the binding cpm when the standard is used for competition. Accordingly, the smaller the value B/Bo (%), the higher is the competitiveness. This value decreases with an increase in the amount of standard.

(c) FH-6 antibody standard (50 µl) prepared in the same manner as in (a), 200 µl of the same buffer as used in (a) and an immobilized bead of Ald-6F were reacted with shaking at room temperature for 2 hours. With addition of 100 µl of $^{125}I$-labeled FH-6 (prepared using the same buffer as in (a), about 20000 cpm), the mixture was further reacted for 1 hour. The bead was thereafter washed. Fig. 5 shows a standard curve similarly obtained.

(d) A mixture was prepared from 100 µl of FH-6 antibody standard prepared in the same manner as in (a), 100 µl of a solution (about 20000 cpm) $^{125}I$-labeled FH-6 in 50 mM PBS buffer (pH 7.4, containing 0.15M NaCl, 0.1% BAS and 0.05% $NaN_3$), 100 µl of Ald-6F as diluted to 1µ g/ml with the same buffer, and 100 µl of the same buffer. The mixture was allowed to stand overnight at 4°C. To the mixture were added 100 µl of normal rat plasma diluted X400 with the above buffer, 100 µl of anti-rat IgG antibody diluted X40 with the above buffer and 200µ l of 10% aqueous solution of polyethylene glycol. The resulting mixture was centrifuged (3000 rpm, 30 minutes). The supernatant was decanted for B/F separation. Fig. 6 shows a standard curve obtained by measuring the sediment.

(2) System for determining Sialyl Le $^x$-i antigen (using Ald-6F as standard)

(a) Solutions of varying concentrations were prepared by dissolving Ald-6F in goat serum to use the solutions in place of Sialyl $Le^x$-i antigen. Each of the solutions (50 µl) and an immobilized bead of FH-6 antibody were reacted in 50 mM citrate buffer (pH 4.5) containing 0.1% BSA and 0.05% $NaN_3$ with shaking at room temperature for two hours. After removing the unreacted substances by washing, 200 µl of a solution of $^{125}I$-labeled FH-6 antibody in the same buffer as above (about 7000 cpm) was added to the reaction mixture. The mixture was reacted with shaking at room temperature for 2 hours. After removing the unreacted substances by washing, the bead was checked for radioactivity by a gamma counter. Fig. 7 shows a standard curve prepared from the result.

(b) In the same manner as in (a), 50 µl of AId-6F standard was reacted with 200 µl of a solution of about 20000 cpm of $^{125}$I-labeled FH-6 in citrate buffer with shaking at room temperature for 2 hours. Further with addition of an insolubilized AId-6F bead, the mixture was subjected to competitive reaction for 1 hour. After washing, the radioactivity of the bead was measured with a gamma counter. Fig. 8 shows a standard curve obtained.

(c) In the same manner as in (a), 50 µl of AId-6F standard was reacted with an immobilized bead of FH-6 in 200 µl of the same citrate buffer as used above which contained $^{125}$I-labeled AId-6F (30000 cpm) with shaking at room temperature for 2 hours to effect competitive reaction. After removing the unreacted substance, the bead was checked for radioactivity by a gamma counter.

(d) In the same manner as in (a), competitive reaction was carried out using 50 µl of solution of AId-6F standard in 200 µl of the above citrate buffer containing $^{125}$I-balelled FH-6 (20000 cpm) with shaking at room temperature for 2 hours. With addition of one bead coated with PC-9 extracts, the mixture was further reacted for 1 hour. After washing, the bead was checked for radioactivity with a gamma counter. Fig. 10 shows a standard curve obtained.

## Test Example 4

(1) Determination of FH-6 antibody in serum

To human serum (50 µl) was added 200 µl of 50 mM PBS (pH 7.4) containing 0.1% BSA and 0.05 $NaN_3$. Four samples were prepared for each of normal persons and cancer patients. An immobilized AId-6F antibody bead was placed into two of the samples, and an immobilized bead of PC-9 extract was placed into the other samples. Each of the samples was then shaken at room temperature for 2 hours for reaction. After the reaction, the unreacted substances were removed by washing with distilled water three times. $^{125}$I-labeled AId-6F antibody (200 µ, 100,000 cpm) was added to the beads of one kind, and 200µ l of $^{125}$I-labeled anti-human IgG (50,000 cpm) was added to the beads of the other kind. Each mixture was then shaken at room temperature for 2 hours for reaction. Subsequently, the unreacted substances were removed by washing with distilled water three times. The beads were thereafter checked for radioactivity with a gamma counter.

Fig. 11 shows the result, which reveals a significant different between 50 cases of normal persons and 10 cases of lung cancer patients.

(2) Detection of cells producing FH-6 antibody

FH-6 hybridoma (control: AH-6 hybridoma) solution having a final concentration of 10 million cells/ml was prepared after washing FH-6 hybridomas with cold PBS three times. The FH-6 and AH-6 hybridoma solutions (50 µl each) and 50 µ of fluorescence-labeled AId-6F (100, 33.3, 11.1 or 3.7 µg/ml) were reacted with ice cooling for 30 minutes. The reaction mixture was washed with cold PBS once, then diluted to 1 ml and thereafter subjected to flow cytometory using a cytometer (Spectran III, product of Ortho Diagnostic Co., Ltd.) under the conditions of excitation at 488 nm and emission at 530 nm. The ratio of FH-6 reactive with fluorescence-labeled antibody AId-6F was compared with that of AH-6.

Fig. 12 shows the result, indicating that anti-idiotype antibody AId-6F reacts only with FH-6 antibody producing cells.

## Test Example 5

The culture supernatant of clone No.OAL-AId-2X42, OAL-AId-2X16, OAL-AId-2X24, OAL-AId-2X26 or OAL-AId-2X50 was diluted (X10, X30, X90, X270, X810 or X2430) with complete RPMI-1640 medium. Tubes each containing 50 µl of each dilution and 200 µl of phosphate buffer (pH 7.4) were prepared. Immobilized beads of various antibodies, BALB/c mouse $IgG_1$, $IgG_2a$, $IgG_2b$, IgM antibody and BSA were placed into the respective tubes in the same manner as in Test Example 1. Each mixture was reacted with shaking at room temperature for 2 hours. The reaction mixture was thereafter treated in the same manner as in Test Example 1 to terminate the reaction and then checked for absorbance at 492 nm.

Fig. 13 shows the result, revealing that Ald-1(2), Ald-2(5), Ald-3(3), Ald-4(4) and Ald-5(1) reacted with the bead of FH-2 only and failed to react with any of the other beads of antibodies and BSA. This indicates that these antibodies are anti-idiotype antibodies having specific reactivity with FH-2.

(2) The culture supernatant (50 µl) of OAL-Ald-2X42, OAL-Ald-2X16, OAL-Ald-2X24, OAL-Ald-2X26 or OAL-Ald-2X50 was reacted with 200 µl (50000 cpm) of $^{125}$I-labeled FH-2 with shanking at room temperature overnight. The reaction mixture (200 µl) was then transferred onto a microplate having PC-7 cells fixed thereto as an antigen, followed by reaction with shaking at room temperature for 90 minutes. Subsequently, the plate was washed with distilled water three times and 200 µl of 2N NaOH was placed on the plate to terminate the antigen-antibody reaction. The radioactivity of the $^{125}$I-labeled FH-2 remaining in the supernatant was measured. In this way, an anti-idiotype antibody was searched for which inhibited antibody-antigen binding.

Fig. 14 shows the result, indicating that Ald-1(2) slightly inhibited binding between FH-2 antibody and antigen and that Ald-2(5), Ald-3(3), Ald-4(4) or Ald-5(1) was anti-idiotype antibody of the type dose-dependently inhibiting the binding.

(3) Dilutions (X1,X3, X9, X27, X81 and X243) of the culture supernatant of anti-idiotype antibody (Ald-1, Ald-2, Ald-3, Ald-4 or Ald-5) having varying concentrations were prepared. Each of the dilutions (50 µl) was added to 200 µl of solution (20000 cpm) of $^{125}$I-labeled anti-idiotype antibody (Ald-4) in 50 mM PBS (pH 7.4) buffer containing 0.1% BSA and 0.05% $NaN_3$. An immobilized bead of FH-2 antibody was placed into the mixture and reacted therewith at room temperature for 90 minutes with shaking. The unreacted substances were then removed by washing with distilled water three time. The bead was then checked for radioactivity with a gamma counter.

Fig. 14 shows the result, indicating that Ald-1(2) differed from the other four anti-idiotype antibodies ((1) to (5)) in epitope.

(4) FH-2 antibody was dissolved in goat serum to varying concentrations to obtain standards. Each standard (50 µl) and one immobilized bead of anti-idiotype antibody (insolubilized Ald-1, Ald-2, Ald-3, Ald-4 or Ald-5 bead) obtained above were added to 200 µl of 50 mM PBS (pH 7.4) buffer containing 0.1% BSA and 0.05% $NaN_3$. The mixture was reacted with shaking at room temperature for 2 hours. After removing the unreacted substances by washing, the reaction mixture was further reacted with 200 µl of a solution (about 100,000 cpm) of $^{125}$I-labeled anti-idiotype antibody (Ald-1, Ald-2, Ald-3, Ald-4 or Ald-5) obtained above in the same buffer as above (with shaking at room temperature for 2 hours). After removing the unreacted substances, the bead was checked for radioactivity with a gamma counter. fig. 16 shows a standard curve prepared from the result.

## Test Example 6

(1) Dilutions (X10, X30, X90, X270, X810 and X2430) were prepared in dissolving the culture supernatant of OAL-Ald-4Y40, OAL-Ald-4Y12 or OAL-Ald-4Y26 in complete RPMI-1640 medium. Each dilution (50 µl) was treated in the same manner as in Test Example 1 to check the supernatant for reactivity with various antibodies.

Fig. 17 shows the result, indicating that Ald-6(1), Ald-7(3) or Ald-8(2) reacted with the immobilized bead of AH-6 antibody only and failed to react with any other immobilized beads of antibodies and BSA. This reveals that these antibodies are anti-idiotype antibodies having specific reactivity with AH-6 antibody.

(2) The culture supernatant (50 µl) of OAL-Ald-4Y40, OAL-Ald-4Y12 or OAL-Ald-4Y26 was reacted with 200 µl (50000 cpm) of $^{125}$I-labeled AH-6 antibody with shaking at room temperature overnight. A polystyrene bead bearing an insolubilized perchloric acid extract of a liver cancer tissue derived from colon cancer was placed as an antigen into the reaction mixture. The mixture was reacted with shaking at room temperature for 90 minutes, and the bead was thereafter washed with distilled water three times. Subsequently, the bead was placed into a new tube, and the $^{125}$I-labeled AH-6 antibody remaining on the bead was checked for radioactivity. In this way, an anti-idiotype antibody was searched for which inhibited antibody-antigen reaction.

Fig. 18 shows the result, indicating that although Ald-6(1) slightly inhibited binding between AH-6 antibody and the antigen, Ald-7(3) or Ald-8(2) was an anti-idiotype antibody of the type dose-dependently inhibit the binding.

(3) Dilutions (X1, X3, X9, X27, X81 and X243) of the culture supernatant of anti-idiotype antibody (AId-6, AId-7 or AId-8) having varying concentrations were prepared. Each of the dilutions (50 µl) was added to 200 µl of solution (20000 cpm) of $^{125}$I-labeled anti-idiotype antibody (AId-7) in the same buffer as used in Test Example 5-(3). An immobilized bead of FH-6 antibody was placed into the mixture and reacted therewith at room temperature for 90 minutes with shaking. The unreacted substances were then removed by washing with distilled water three time. The bead was then checked for radioactivity with a gamma counter.

Fig. 19 shows the result, indicating that AId-6(1) differs from AId-6(3) or AId-8(2) in epitope.

(4) In the same manner as in Test Example 5-(4), 50µ l of standard solution of AH-6 antibody, an immobilized bead of anti-idiotype antibody (insolubilized AId-6, AId-7 or AId-8 bead) obtained above and 200 µl of the about buffer were reacted with shaking at room temperature for 2 hours. After removing the unreacted substances by washing, the reaction mixture was further reacted with 200 µl of a solution (about 100,000 cpm) of $^{125}$I-labeled anti-idiotype antibody (AId-6, AId-7 or AId-8) obtained above in the same buffer as above (with shaking at room temperature for 2 hours). After removing the unreacted substances, the bead was checked for radioactivity with a gamma counter. Fig. 20 shows a standard curve prepared from the result.

Test Example 7

(1) Dilutions (X10, X30, X90, X270, X810 and X2430) were prepared in dissolving the culture supernatant of OAL-AId-19A4, OAL-AId-19A7 or OAL-AId-19A10 in complete RPMI-1640 medium. Each dilution (50 µl) was treated in the same manner as in Test Example 1-(1) to check the supernatant for reactivity with various antibodies.

Fig. 21 shows the result, indicating that AId-9(1), AId-10(3) or AId-11(2) reacted with the immobilized bead of CA19-9 antibody only and failed to react with any other immobilized beads of antibodies and BSA. This reveals that these antibodies are anti-idiotype antibodies having specific reactivity with CA19-9 antibody.

(2) The culture supernatant (50 µl) of OAL-AId-19A4, OAL-AId-19A7 or OAL-AId-19A10 was reacted with 200 µl (50000 cpm) of $^{125}$I-labeled AH-6 antibody with shaking at room temperature overnight. A polystyrene bead bearing a glycoprotein as insolubilized thereon was placed into the mixture was an antigen. The glycoprotein was prepared by subjecting KATO-III cells to extraction with perchloric acid and purifying the extract by affinity chromatography using PNA Sepharose CL-4B. The mixture was reacted at room temperature for 90 minutes, and the bead was thereafter washed with distilled water three times. Subsequently, the bead was placed into a new tube, and the $^{125}$I-labeled CA19-9 antibody remaining on the bead was checked for radioactivity. In this way, an anti-idiotype antibody was searched for which inhibited antibody-antigen reaction.

Fig. 22 shows the result, indicating that AId-9(1) was an anti-idiotype antibody of the type dose-dependently inhibiting antibody-antigen binding and that AId-10(3) and AId-11(2) were anti-idiotype antibodies not inhibiting the binding.

(3) Dilutions (X1, X3, X9, X27, X81 and X243) of the culture supernatant of anti-idiotype antibody (AId-9, AId-10 or AId-11) having varying concentrations were prepared. Each of the dilutions (50 µl) was added to 200µ l of solution (20000 cpm) of $^{125}$I-labeled anti-idiotype antibody (AId-9) in 50 mM PBS (pH 7.4) buffer containing 0.1% BSA and 0.05% $NaN_3$. An immobilized bead of CA19-9 antibody was placed into the mixture and reacted therewith at room temperature for 90 minutes with shaking. The unreacted substances were then removed by washing with distilled water three time. The bead was then checked for radioactivity with a gamma counter.

Fig. 23 shows the result, indicating that AId-9(1) differs from AId-10(3) or AId-11(2) in epitope.

(4) In the same manner as above, 50 µl of standard solution of CA19-9 antibody, an immobilized bead of anti-idiotype antibody (insolubilized AId-9, AId-10 or AId-11 bead) obtained above and 200 µl of the above buffer were reacted with shaking at room temperature for 2 hours. After removing the unreacted substances by washing, the reaction mixture was further reacted with 200 µl of a solution (about 100,000 cpm) of $^{125}$I-labeled anti-idiotype antibody (AId-9, AId-10 or AId-11) obtained above in the same buffer as above (with shaking at room temperature for 2 hours). After removing the unreacted substances, the bead was checked for radioactivity with a gamma counter. Fig. 24 shows a standard curve prepared from the result.

Test Example 8

(1) Dilutions (X30, X90, X270, X810, X2430 and X7290) were prepared by dissolving the culture supernatant of OAL-Ald-7A1, OAL-Ald-7A2 or OAL-Ald-7A3 in complete RPMI-1640 medium. Each dilution (200 μl) was treated in the same manner as in Test Example 1 to check the supernatant for reactivity with various antibodies.

Fig. 25 shows the result, indicating that Ald-7A1, Ald-7A2 or Ald-7A3 reacted with the immobilized bead of FH-7 antibody only and failed to react with any other immobilized beads of antibodies and BSA. This reveals that these antibodies are anti-idiotype antibodies having specific reactivity with FH-7 antibody.

(2) The culture supernatant (50 μl) of OAL-Ald-7A1, OAL-Ald-7A2 or OAL-Ald-7A3 was reacted with 200 μl (100000 cpm) of $^{125}I$-labeled FH-7 antibody with shaking at room temperature for 1 hour. Subsequently, an immobilized bead of glycoprotein derived from meconium and obtained by extraction and purification in the same manner as in Example 5-(1) was added to the reaction mixture as an antigen. The mixture was reacted with shaking at room temperature for 1 hour. The bead was thereafter washed with distilled water three times. Subsequently, the bead was placed into a new tube, and the $^{125}I$-labeled FH-7 antibody remaining on the bead was checked for radioactivity. In this way, an anti-idiotype antibody was searched for which inhibited antibody-antigen reaction.

Fig. 26 shows the result, indicating that Ald-7A3 did not inhibit binding betwen FH-7 antibody and the antigen, and that Ald-7A1 or Ald-7A2 was an anti-idiotype antibody of the type dose-dependently inhibit the binding.

Test Example 9

(1) Dilutions (X30, X90, X270, X810, X2430 and X7290) were prepared by dissolving the culture supernatant of OAL-Ald-9A1 in complete RPMI-1640 medium. Each dilution (200 μl) was treated in the same manner as in Test Example 1 to check the supernatant for reactivity with various antibodies.

Fig. 27 shows the result, indicating that Ald-9A1 reacted with the immobilized bead of FH-9 antibody only and failed to react with any other immobilized beads of antibodies and BSA. This reveals that the antibody is an anti-idiotype antibody having specific reactivity with FH-9 antibody.

(2) The culture supernatant (50 μl) of OAL-Ald-9A1, was reacted with 200 μl (100000 cpm) of $^{125}I$-labeled FH-9 antibody with shaking at room temperature overnight. The reaction mixture was then transferred onto a plate having PC-7 fixed thereto and seving as an antigen, followed by reaction with shaking at 25°C for 1 hour. The resulting reaction plate mixture was washed with distilled water three times. Subsequently, 200 μl of 2N NaOH was placed onto the plate to dissolve out the antigen with shaking at 25°C for 30 minutes. The resulting mixture was then placed into a new tube and checked for the radioactivity of the $^{125}I$-labeled FH-9 antibody contained therein to identify an anti-idiotype antibody inhibiting antibody-antigen binding.

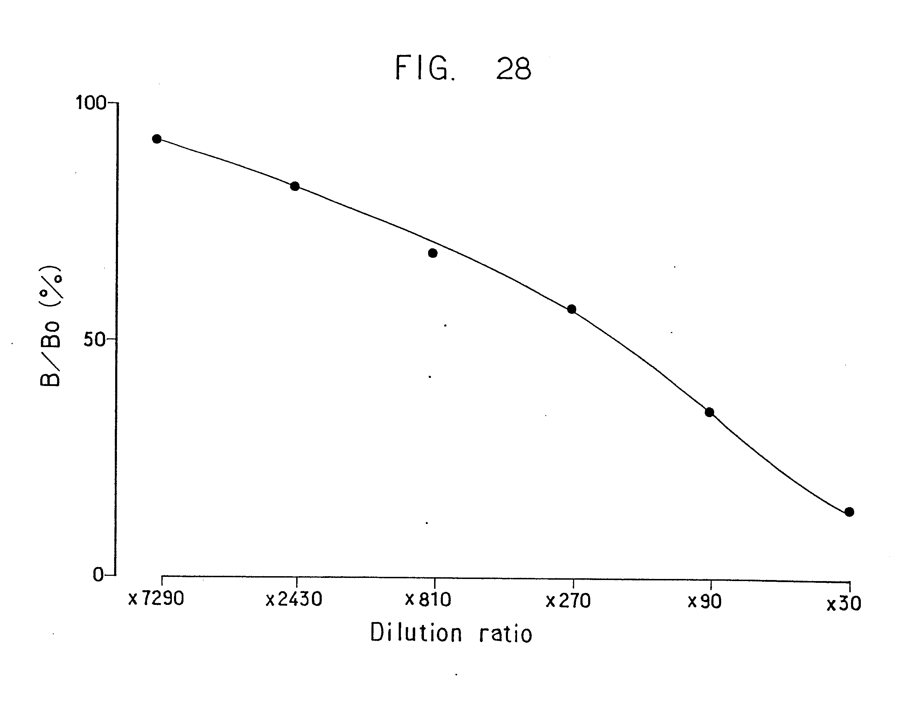

Fig. 28 shows the result, indicating that Ald-9A1 was an anti-idiotype antibody of the type dose-dependently inhibiting binding between FH-9 antibody and the antigen.

## Claims

1. An anti-idiotype antibody to a specific antibody recognizing a carbohydrate linkage selected from the group consisting of
the formula
NeuAcα2→3Galβ1→4GlcNAc(3←α1Fuc)β1→3Galβ1→4GlcNAc(3←α1Fuc)β1→3Galβ1→4Glc
the formula
Galβ1→4GlcNAc(3←α1Fuc)β1→3Galβ1→,
the formula
Fucα1→2Galβ1→4GlcNAc(3←α1Fuc)β1→3Galβ1→,
the formula
NeuAcα2→3Galβ1→3GlcNAc(4←α1Fuc)β1→3Galβ1→,
the formula
NeuAcα2→3Galβ1→3GlcNAc(4←α1Fuc)(6←α2NeuAc)β1→3Galβ1→ and
the formula
NeuAcα2→3Galβ1→3GlcNAc(6←α2NeuAc)β1→3Galβ1→.

2. An anti-idiotype antibody as defined in claim 1 wherein the specific antibody is FH-6, FH-2, AH-6, CA19-9, FH-7 or FH-9.

3. An anti-idiotype antibody as defined in claim 2 wherein said specific antibody is FH-6.

4. An anti-idiotype antibody is defined in claim 2 wherein said specific antibody is FH-2.

5. An anti-idiotype antibody as defined in claim 2 wherein said specific antibody is AH-6.

6. An anti-idiotype antibody as defined in claim 2 wherein said specific antibody is CA19-9.

7. An anti-idiotype antibody as defined in claim 2 wherein said specific antibody is FH-7.

8. An anti-idiotype antibody as defined in claim 2 wherein said specific antibody is FH-9.

9. An anti-idiotype antibody as defined in claim 1, which inhibits the reaction between said carbohydrate linkage and said specific antibody.

10. An anti-idiotype antibody as defined in claim 1, which does not inhibit the reaction between said carbohydrate linkage and said specific antibody.

11. A process for preparing an anti-idiotype antibody characterized by using as an immunizing antigen an specific antibody recognizing a carbohydrate linkage represented by
the formula
NeuAcα2→3Galβ1→4GlcNAc(3←α1Fuc)β1→3Galβ1→4GlcNAc(3←α1Fuc)β1→3Galβ1→4Glc-,
the formula
Galβ1→4GlcNAc(3←α1Fuc)β1→3Galβ1→,
the formula
Fucα1→2Galβ1→4GlcNAc(3←α1Fuc)β1→3Galβ1→,
the formula
NeuAcα2→3Galβ1→3GlcNAc(4←α1Fuc)β1→3Galβ1→,
the formula
NeuAcα2→3Galβ1→3GlcNAc(4←α1Fuc)(6←α2NeuAc)β1→3Galβ1→ or
the formula
NeuAcα2→3Galβ1→3GlcNAc(6←α2NeuAc)β1→3Galβ1→.

12. A process as defined in claim 11 characterized by fusing plasmacyte cells of a mammal immunized with the specific antibody with plasmacytoma cells of a mammal to prepare hybridoma cells, selecting a clone producing the antibody from the hybridoma cells and obtained the desired anti-idiotype monoclonal antibody from the clone.

13. A process as defined in claim 11 or 12 wherein the specific antibody is FH-6, FH-2, AH-6, CA19-9, FH-7 or FH-9.

14. A process as defined in claim 12 wherein said hybridoma cell is the hybrid cell line ATCC No.HB 9207.

15. A process as defined in claim 12 wherein said hybridoma cell is the hybrid cell line ATCC No.HB 9447.

16. A process as defined in claim 12 wherein said hybridoma cell is the hybrid cell line ATCC No.HB 9450.

17. A process as defined in claim 12 wherein said hybridoma cell is the hybrid cell line ATCC No.HB 9461.

18. A process as defined in claim 12 wherein said hybridoma cell is the hybrid cell line ATCC No.HB 9448.

19. A process as defined in claim 12 wherein said hybridoma cell is the hybrid cell line FERM BP-1522.

20. A process as defined in claim 12 wherein said hybridoma cell is the hybrid cell line FERM BP-1523.

21. A method of determining a specific antibody in a sample comprising the steps of contacting the sample with the anti-idiotype antibody of claim 1 and testing for reactivity, said specific antibody recognizing a carbohydrate linkage selected from the group consisting of
the formula
NeuAcα2→3Galβ1→4GlcNAc(3←α1Fuc)β1→3Galβ1→4GlcNAc(3←α1Fuc)β1→3Galβ1→4Glc-,
the formula
Galβ1→4GlcNAc(3←α1Fuc)β1→3Galβ1→,
the formula
Fucα1→2Galβ1→4GlcNAc(3←α1Fuc)β1→3Galβ1→,
the formula
NeuAcα2→3Galβ1→3GlcNAc(4←α1Fuc)β1→3Galβ1→,
the formula

NeuAcα2→3Galβ1→3GlcNAc(4←α1Fuc)(6←α2NeuAc)β1→3Galβ1→ and
the formula
NeuAcα2→3Galβ1→3GlcNAc(6←α2NeuAc)β1→3Galβ1→.

22. A method as defined in claim 21 wherein said sample is a body fluid.

23. A method as defined in claim 21 wherein said sample is an antibody-producing cell.

24. A cancer diagnosing kit comprising the anti-idiotype antibody of claim 1.

25. In an immunoassay for the detection of a cancer antigen having a carbohydrate linkage selected from the group consisting of
the formula
NeuAcα2→3Galβ1→4GlcNAc(3←α1Fuc)β1→3Galβ1→4GlcNAc(3←α1Fuc)β1→3Galβ1→4Glc-,
the formula
Galβ1→4GlcNAc(3←α1Fuc)β1→3Galβ1→,
the formula
Fucα1→2Galβ1→4GlcNAc(3←α1Fuc)β1→3Galβ1→,
the formula
NeuAcα2→3Galβ1→3GlcNAc(4←α1Fuc)β1→3Galβ1→,
the formula
NeuAcα2→3Galβ1→3GlcNAc(4←α1Fuc)(6←α2NeuAc)β1→3Galβ1→ and
the formula
NeuAcα2→3Galβ1→3GlcNAc(6←α2NeuAc)β1→3Galβ1→
in sample on the basis of a selective immunological reaction with a specific antibody recognizing said carbohydrate linkage, the improvement comprising employing the anti-idiotype antibody of claim 9 as a purified antigen.

26. In an immunoassay for the detection of a cancer antigen having a carbohydrate linkage selected from the group consising of
the formula
NeuAcα2→3Galβ1→4GlcNAc(3←α1Fuc)β1→3Galβ1→4GlcNAc(3←α1Fuc)β1→3Galβ1→4Glc-,
the formula
Galβ1→4GlcNAc(3←α1Fuc)β1→3Galβ1→,
the formula
Fucα1→2Galβ1→4GlcNAc(3←α1Fuc)β1→3Galβ1→,
the formula
NeuAcα2→3Galβ1→3GlcNAc(4←α1Fuc)β1→3Galβ1→,
the formula
NeuAcα2→3Galβ1→3GlcNAc(4←α1Fuc)(6←α2NeuAc)β1→3Galβ1→ and
the formula
NeuAcα2→3Galβ1→3GlcNAc(6←α2NeuAc)β1→3Galβ1→
in a sample on the basis of a selective immunolgoical reaction with a specific antibody recognizing said carbohydrate linkage, the improvement comprising employing the anti-idiotype antibody of claim 10 as a second antibody to said specific antibody.

FIG. 1

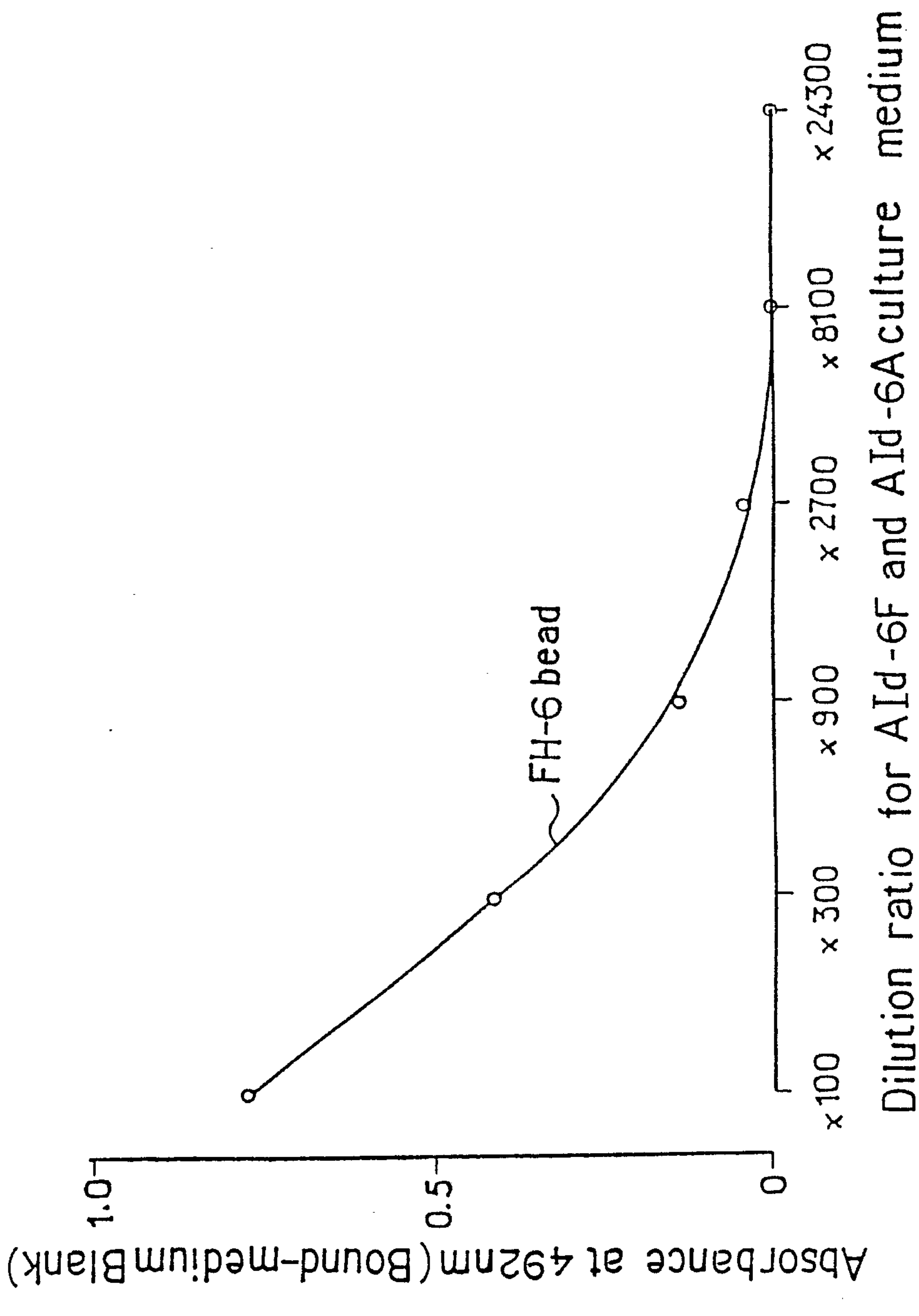
FH-6 bead
Absorbance at 492nm (Bound-medium Blank)
1.0
0.5
0
x100
x 300
x 900
x 2700
x 8100
x 24300
Dilution ratio for AId-6F and AId-6A culture medium

FIG. 2

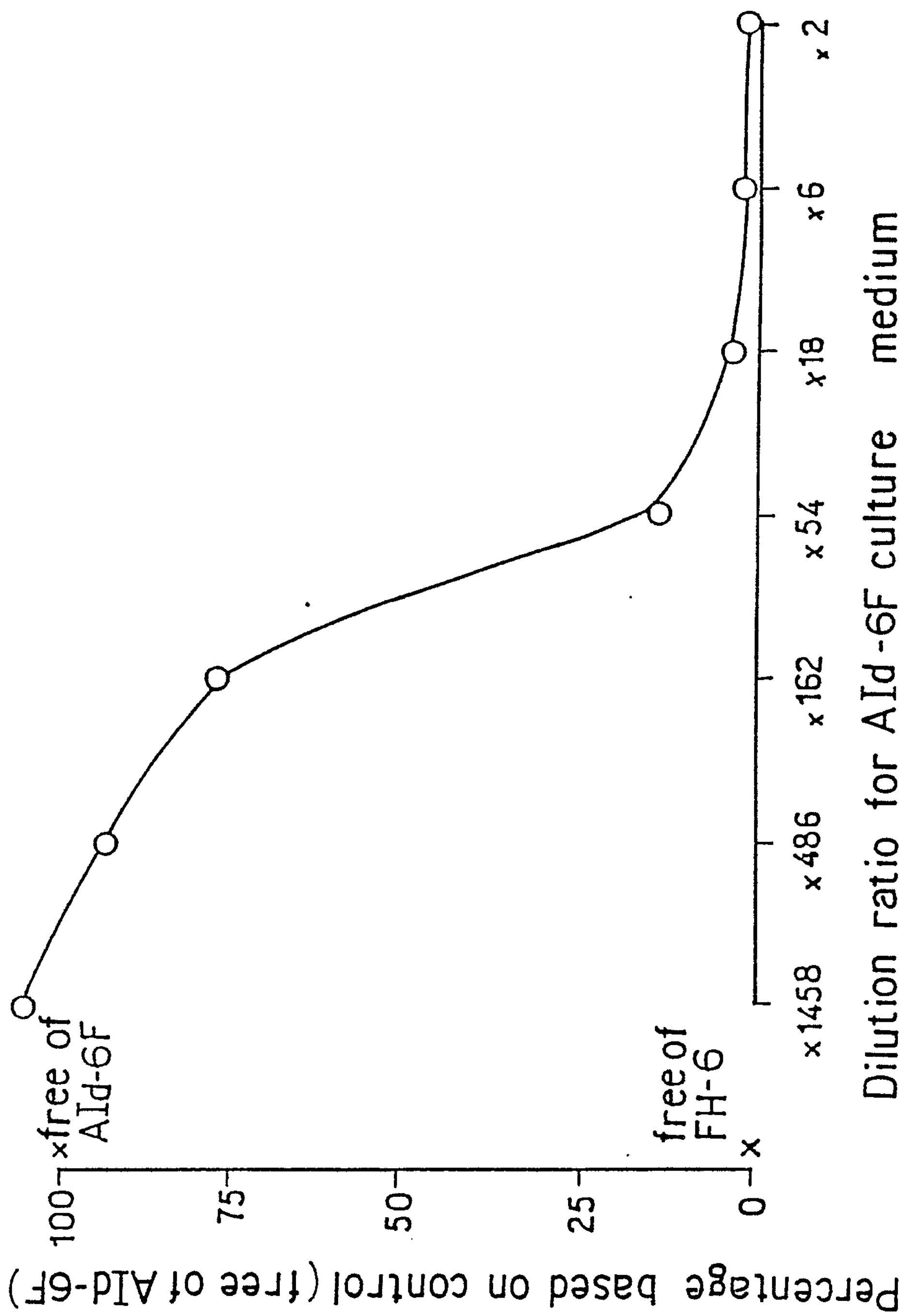
Percentage based on control (free of AId-6F)
100
75
50
25
0
×free of AId-6F
×free of FH-6
x1458
x486
x162
x54
x18
x6
x2
Dilution ratio for AId-6F culture medium

FIG. 3

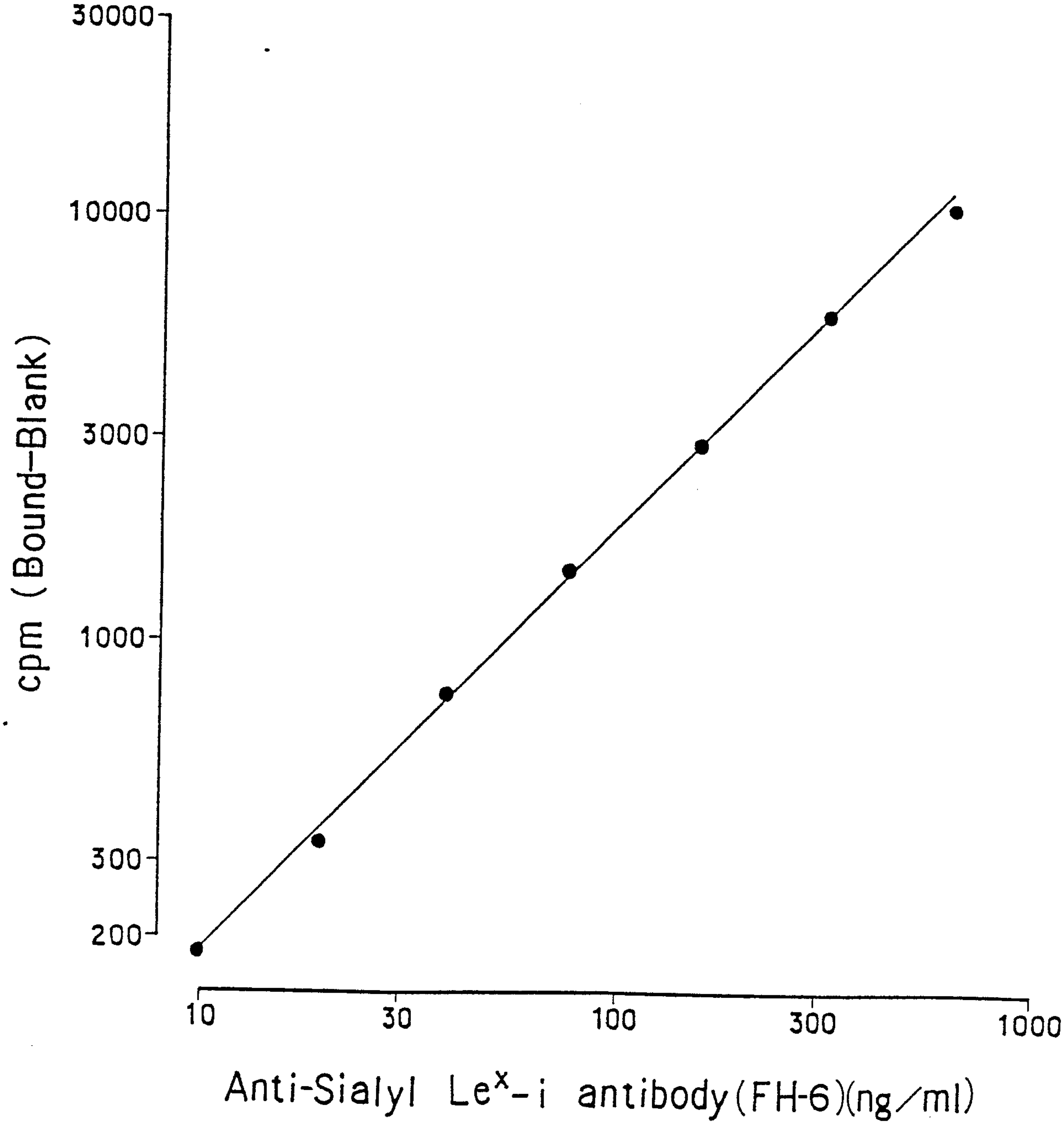
30000
10000
3000
1000
300
200
cpm (Bound-Blank)
10
30
100
300
1000
Anti-Sialyl Le$^{x}$-i antibody(FH-6)(ng/ml)

FIG. 4

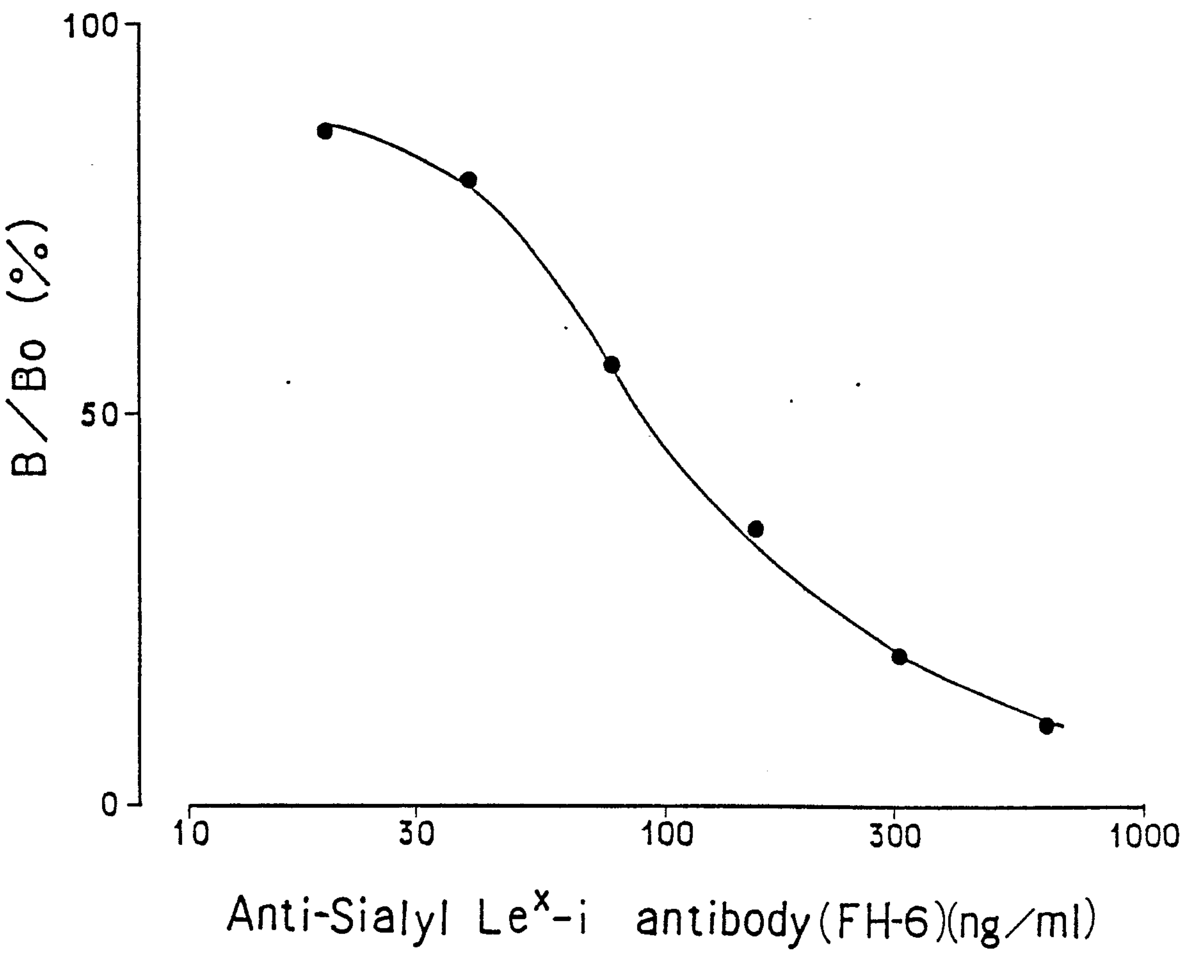
100
50
0
B/Bo (%)
10
30
100
300
1000
Anti-Sialyl Le^x-i antibody(FH-6)(ng/ml)

FIG. 5

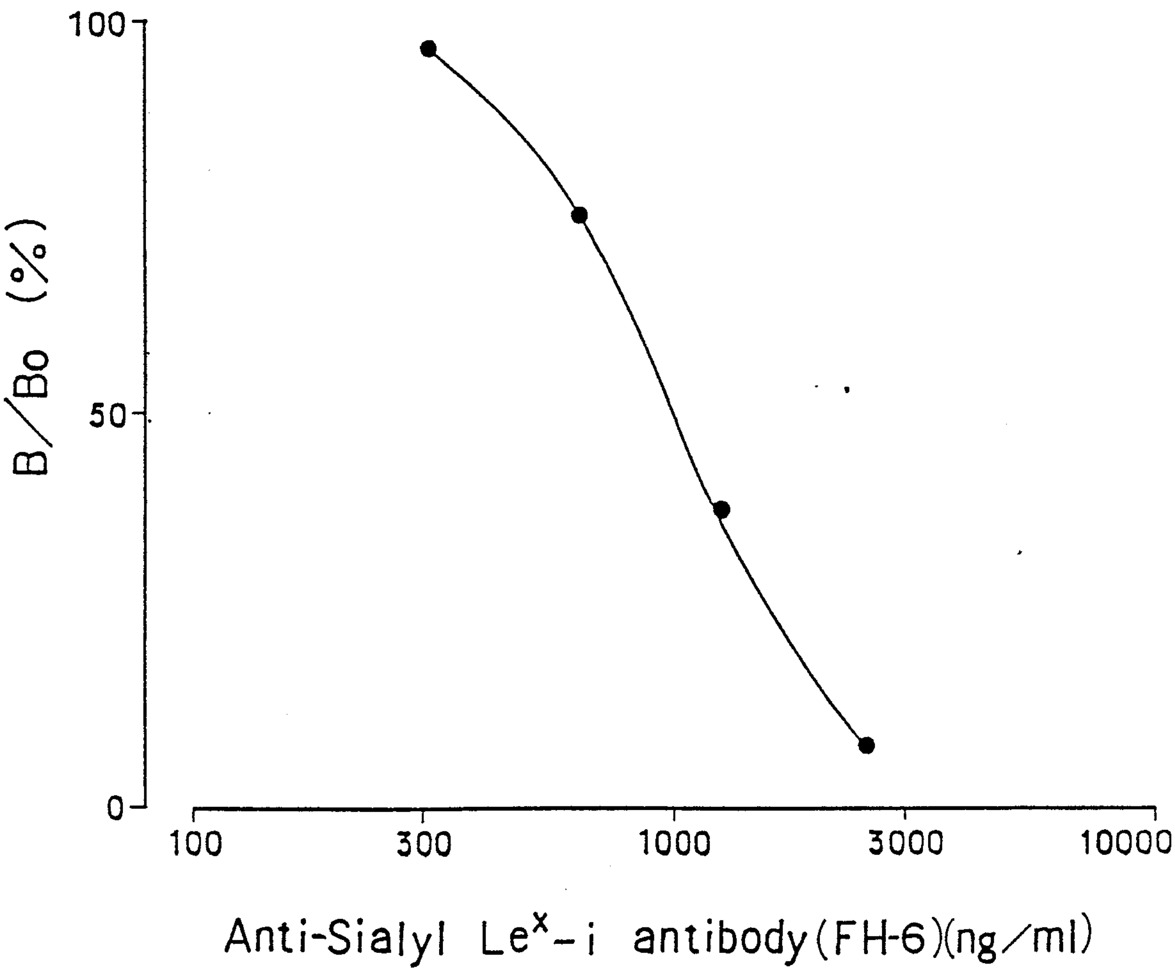
100
50
0
B/Bo (%)
100
300
1000
3000
10000
Anti-Sialyl Le^x-i antibody(FH-6)(ng/ml)

FIG. 6

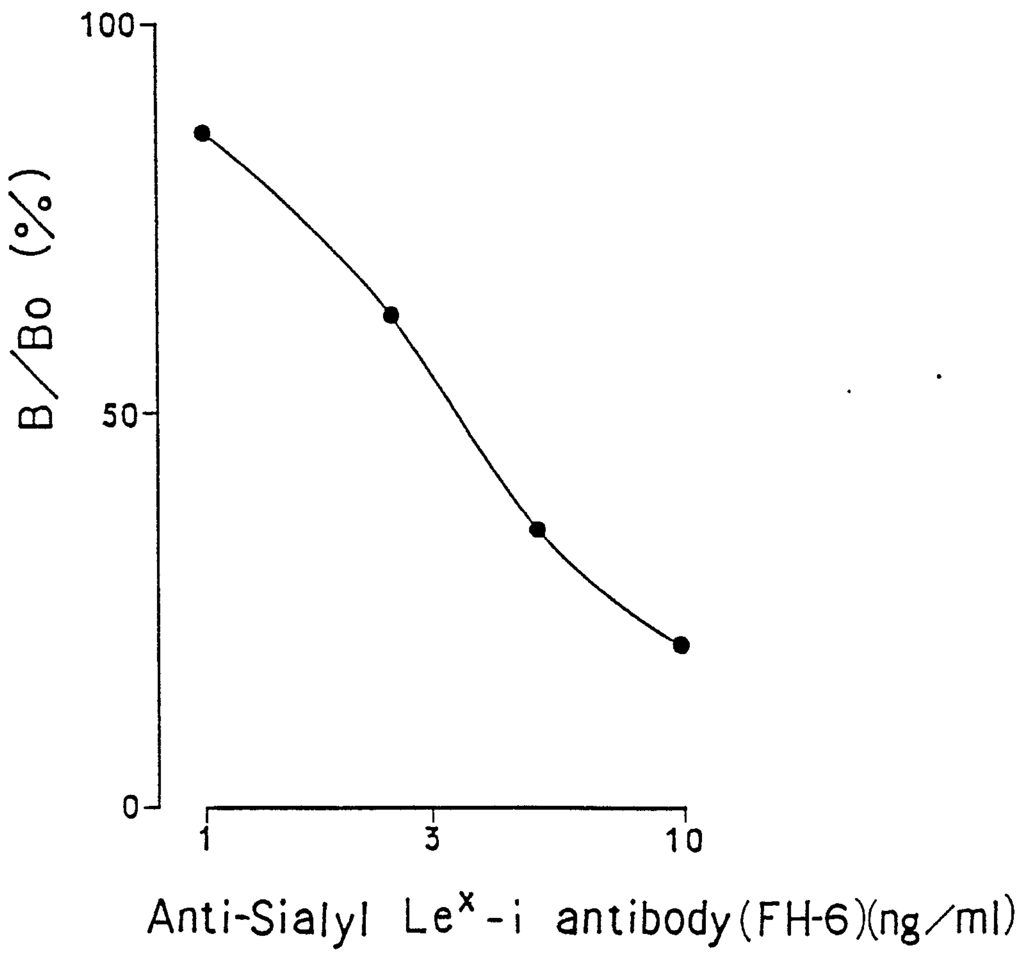

FIG. 7

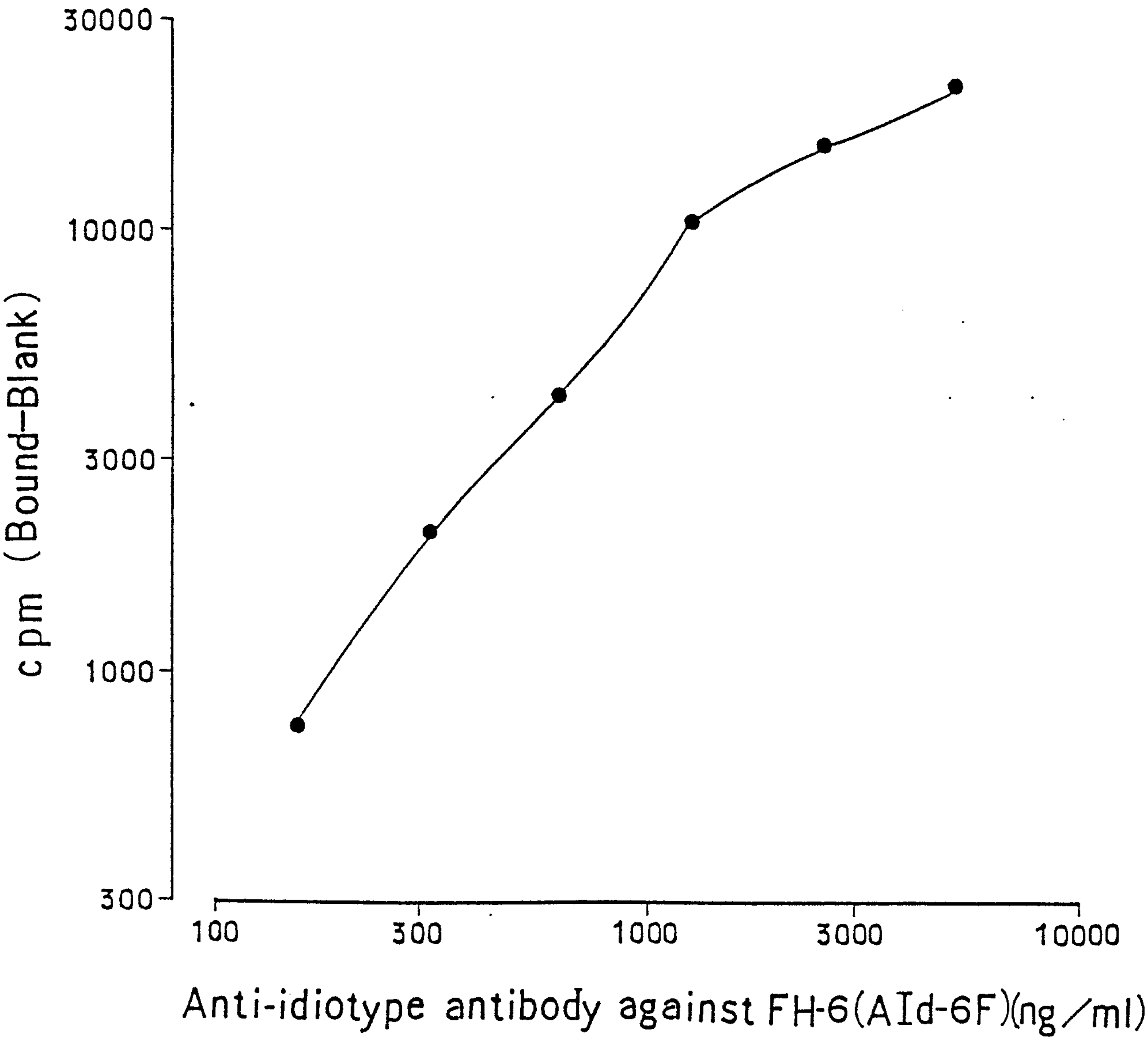
30000
10000
3000
1000
300
cpm (Bound-Blank)
100
300
1000
3000
10000
Anti-idiotype antibody against FH-6(AId-6F)(ng/ml)

FIG. 8

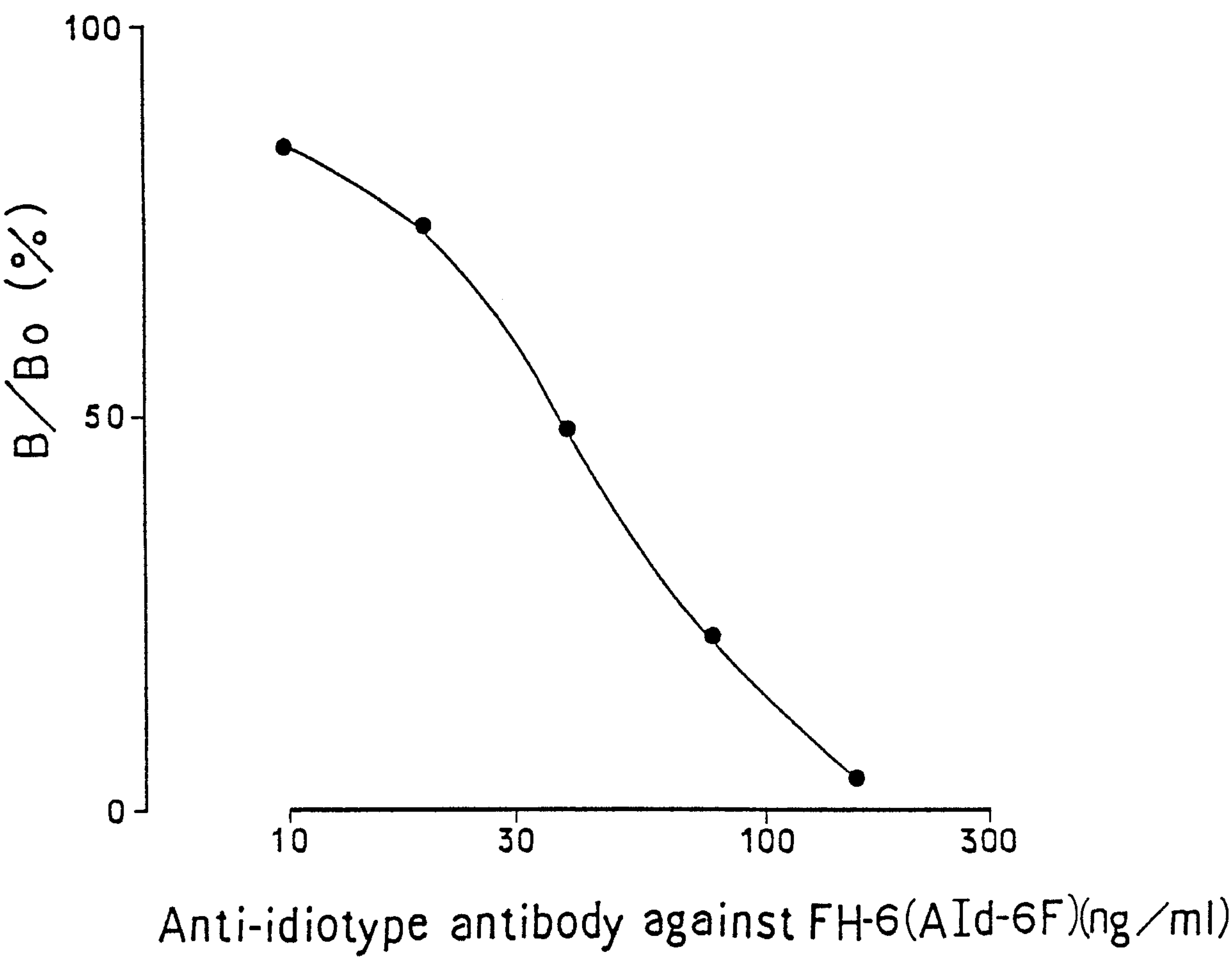
100
50
0
B/Bo (%)
10
30
100
300
Anti-idiotype antibody against FH-6(AId-6F)(ng/ml)

FIG. 9

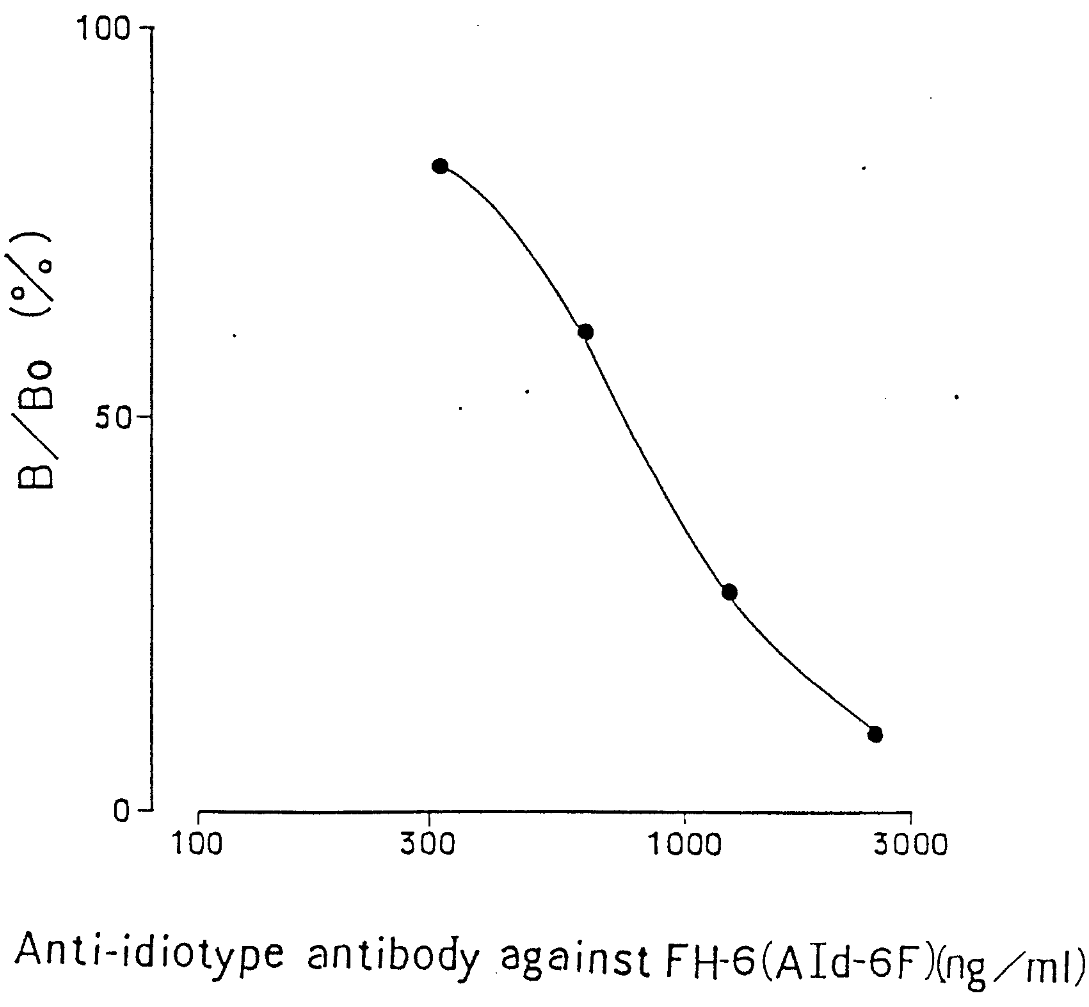

100
50
0
B/Bo (%)
100
300
1000
3000
Anti-idiotype antibody against FH-6(AId-6F)(ng/ml)

FIG. 10

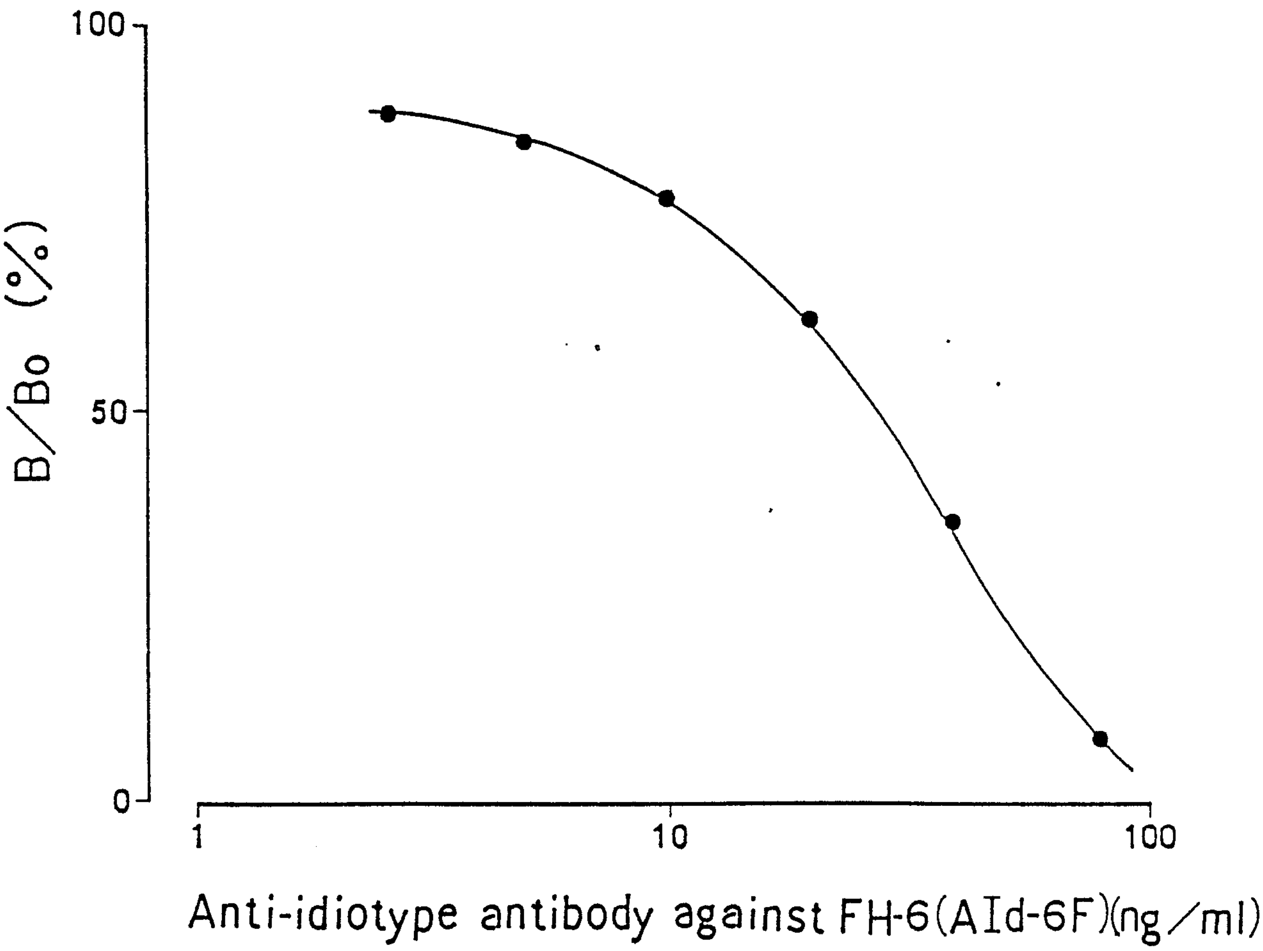
100
50
0
B/Bo (%)
1
10
100
Anti-idiotype antibody against FH-6(AId-6F)(ng/ml)

FIG. 11

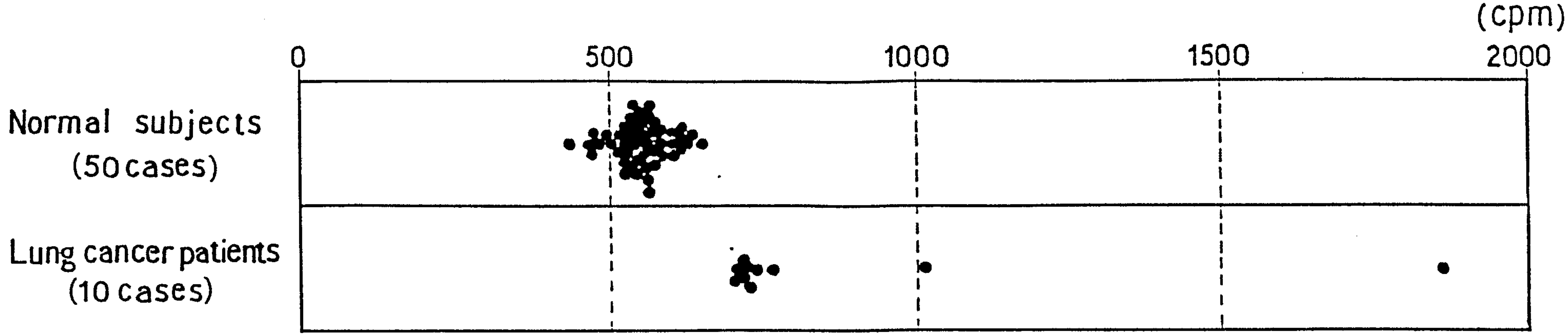
(cpm)
0
500
1000
1500
2000
Normal subjects
(50 cases)
Lung cancer patients
(10 cases)

FIG. 12

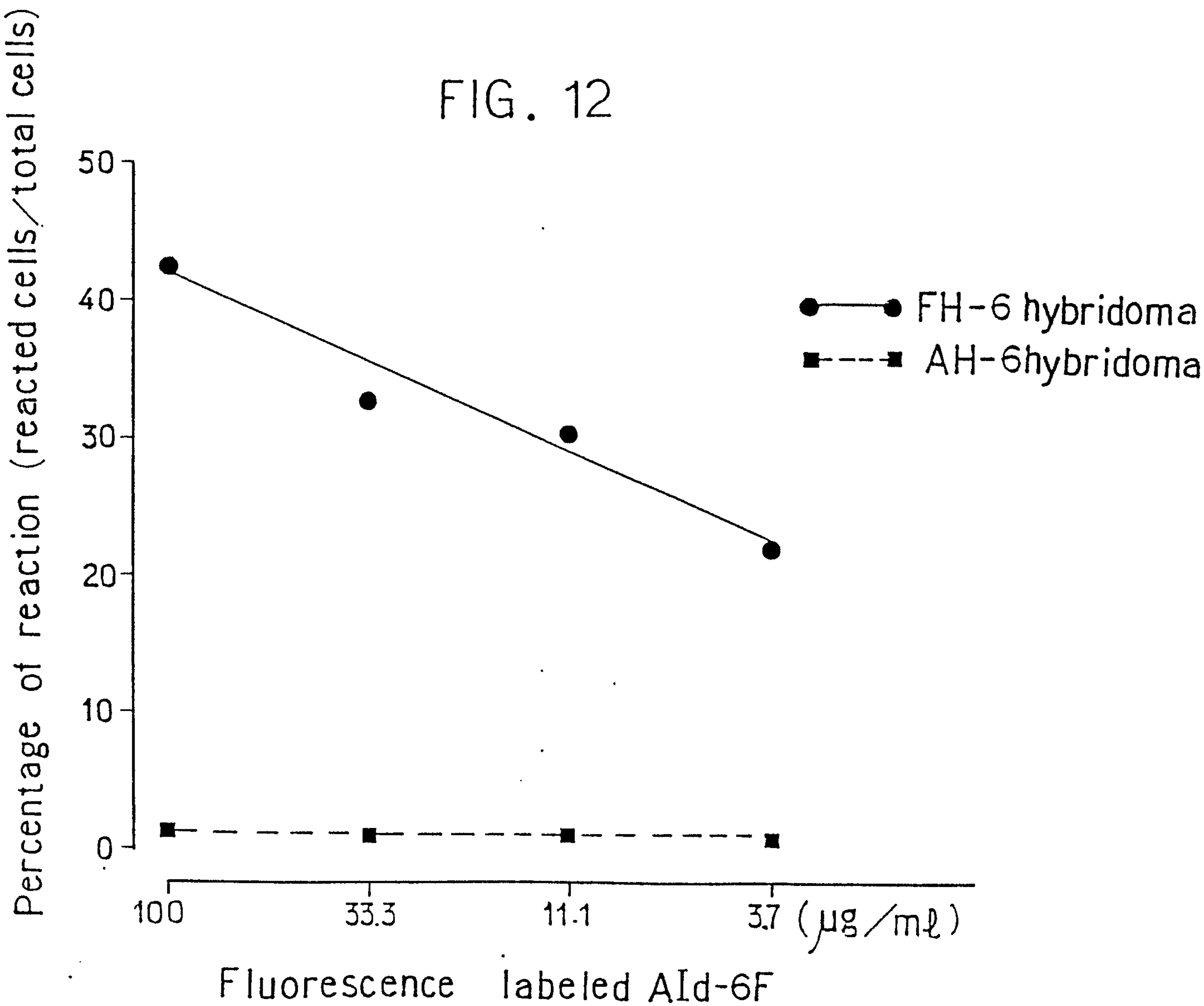
Percentage of reaction (reacted cells/total cells)
50
40
30
20
10
0
100
33.3
11.1
3.7 (μg/mℓ)
Fluorescence labeled AId-6F
FH-6 hybridoma
AH-6hybridoma

FIG. 13

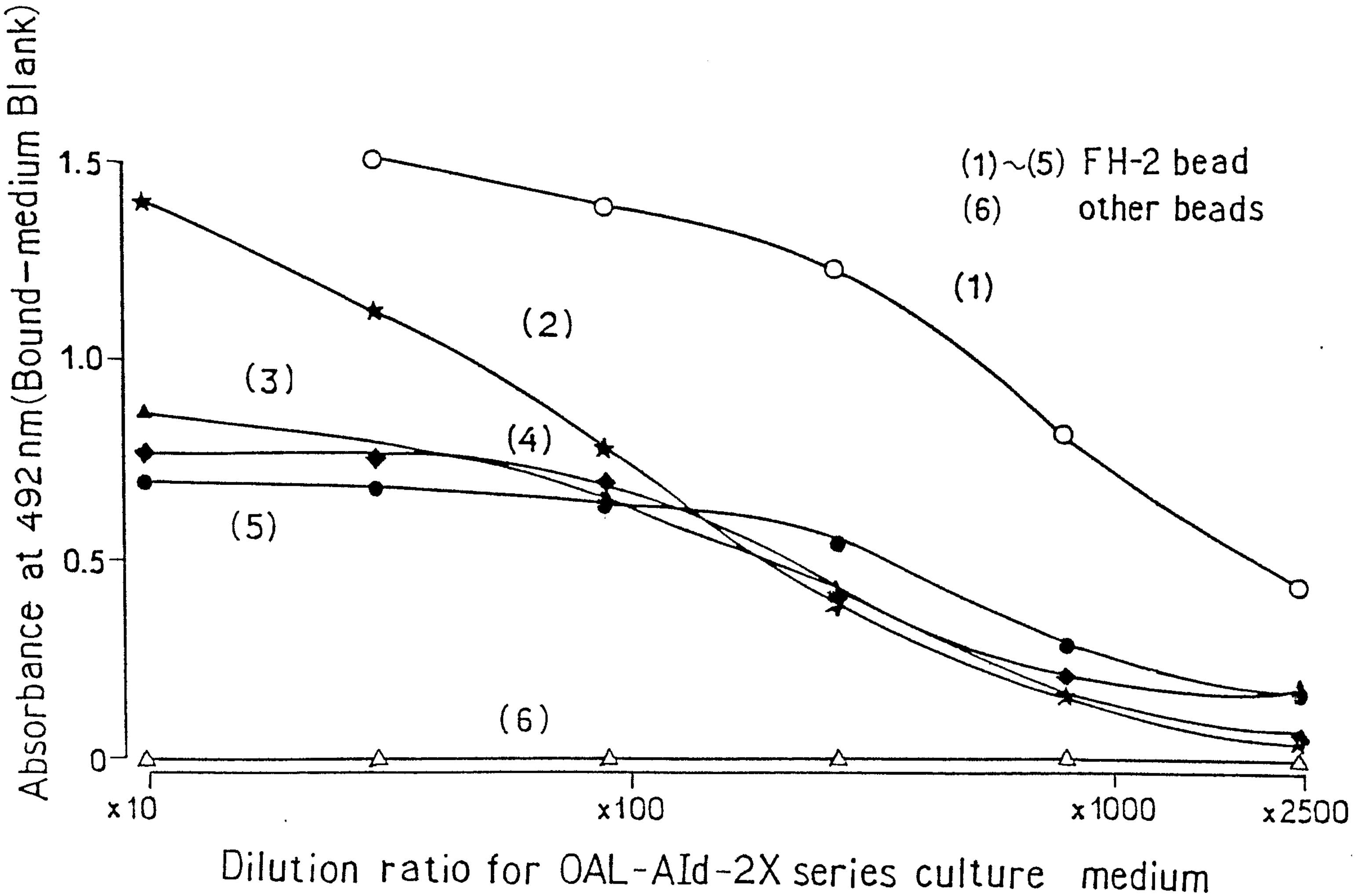
(1)~(5) FH-2 bead
(6) other beads
(1)
(2)
(3)
(4)
(5)
(6)
1.5
1.0
0.5
0
Absorbance at 492 nm (Bound−medium Blank)
x10
x100
x1000
x2500
Dilution ratio for OAL-AId-2X series culture medium

FIG. 14

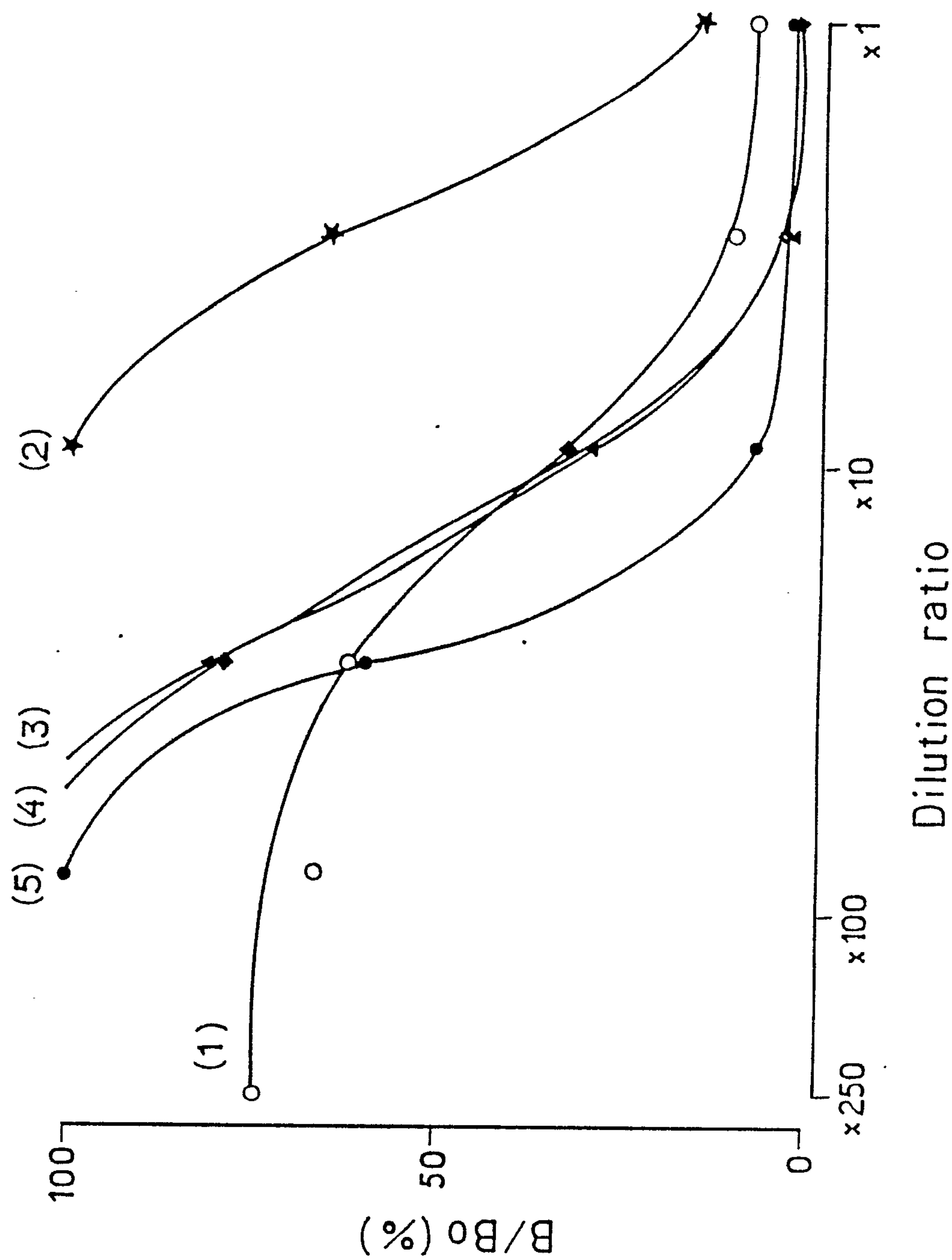

FIG. 15

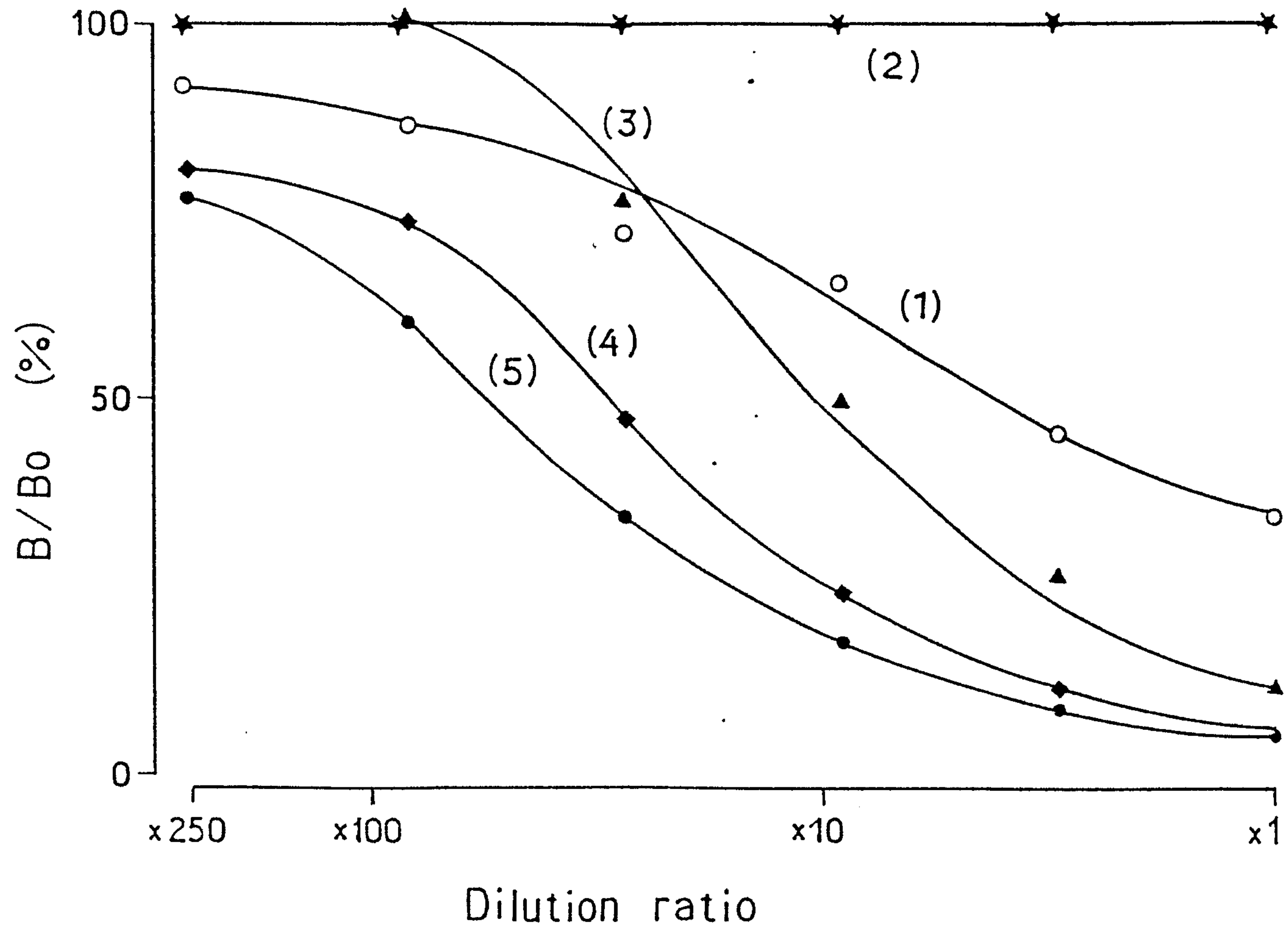

FIG. 16

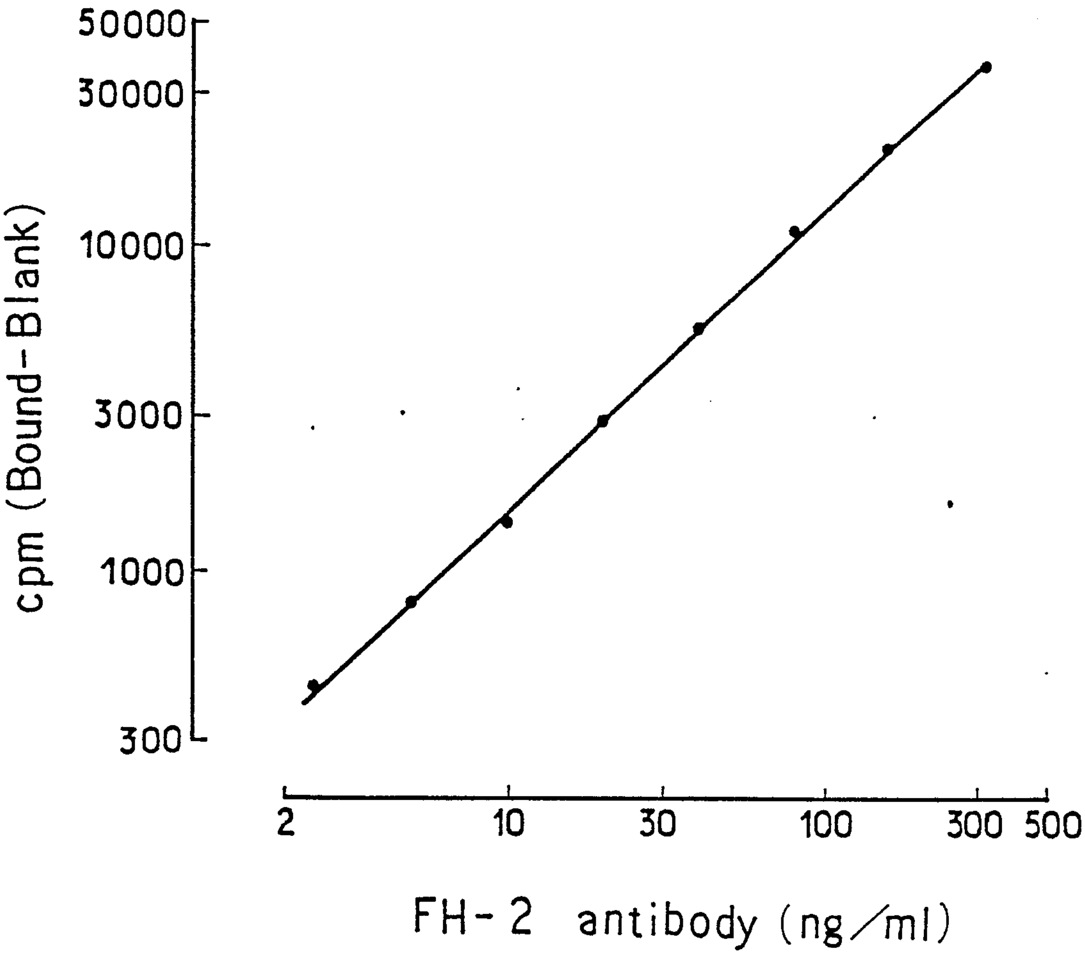
50000
30000
10000
3000
1000
300
cpm (Bound-Blank)
2
10
30
100
300
500
FH-2 antibody (ng/ml)

FIG. 17

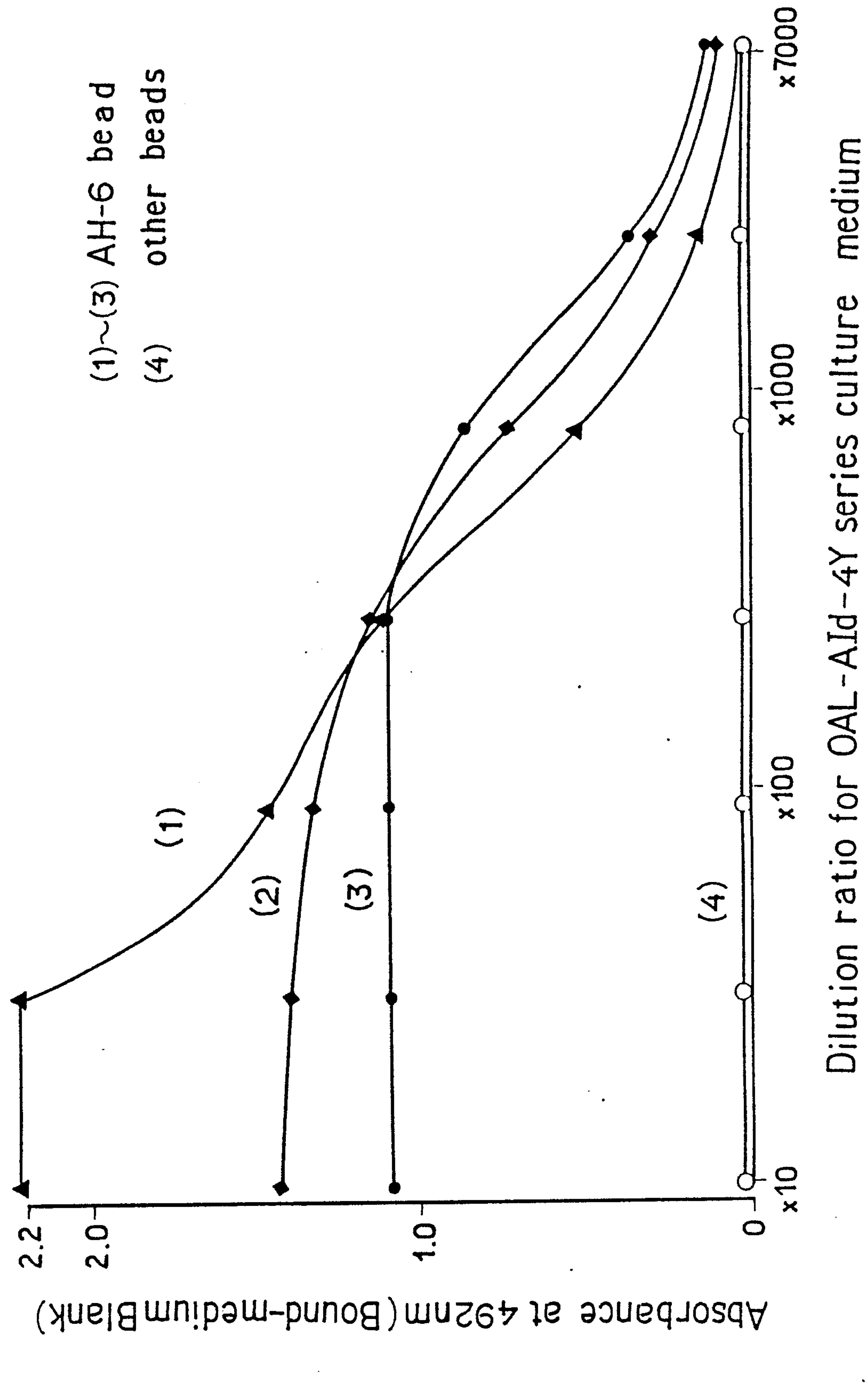
(1)~(3) AH-6 bead
(4) other beads
(1)
(2)
(3)
(4)
2.2
2.0
1.0
0
x10
x100
x1000
x7000
Absorbance at 492nm (Bound-medium Blank)
Dilution ratio for OAL-AId-4Y series culture medium

FIG. 18

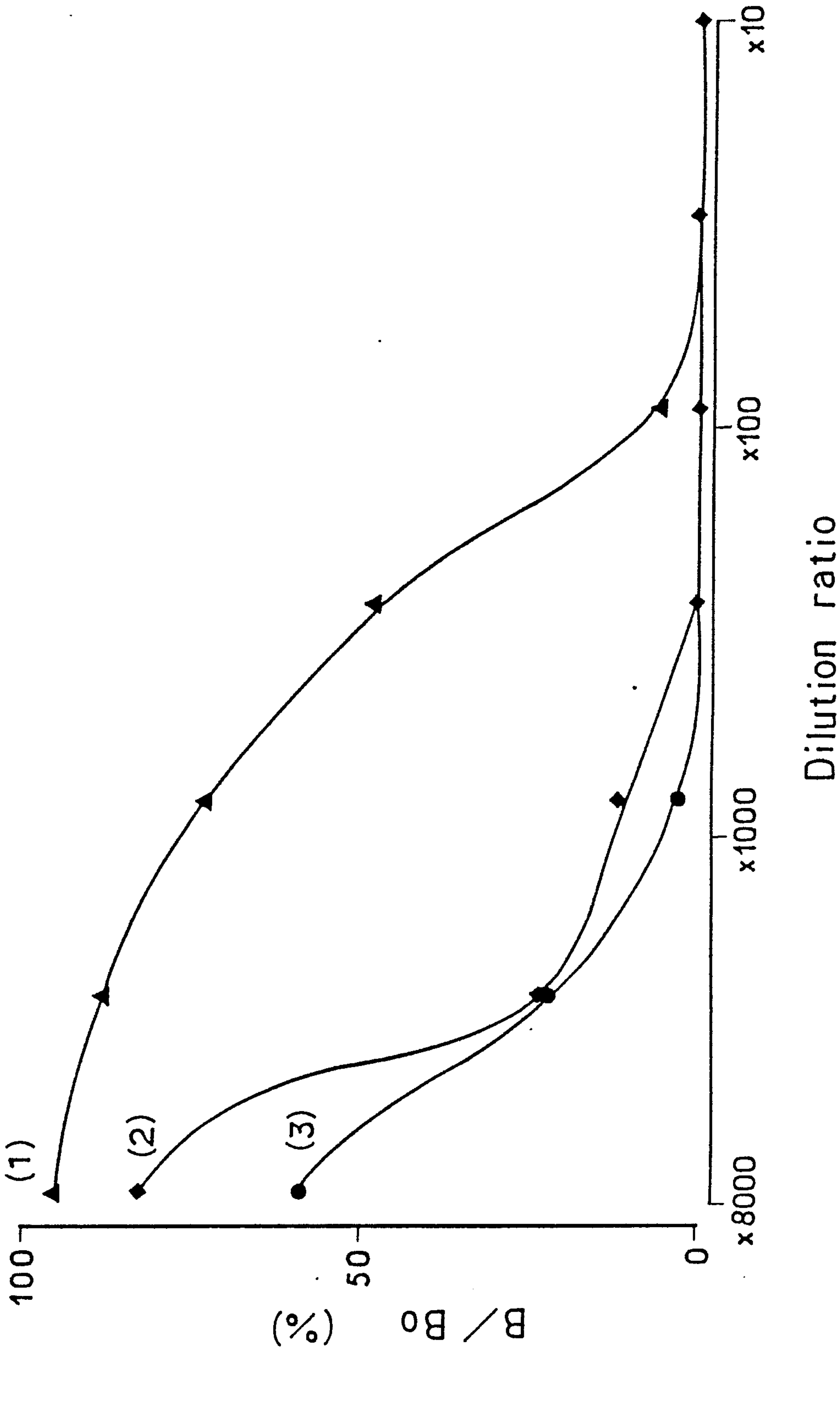

FIG. 19

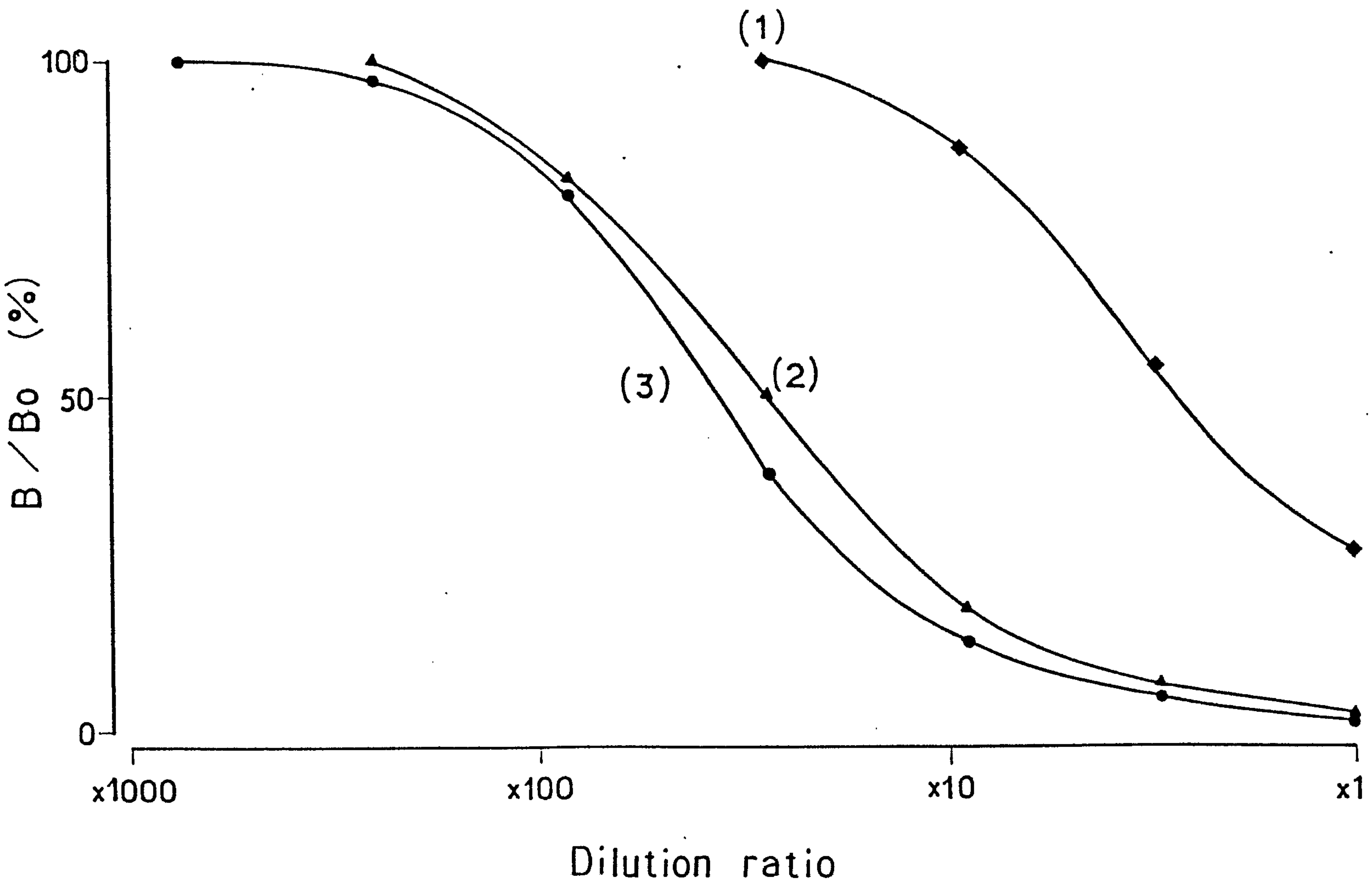

FIG. 20

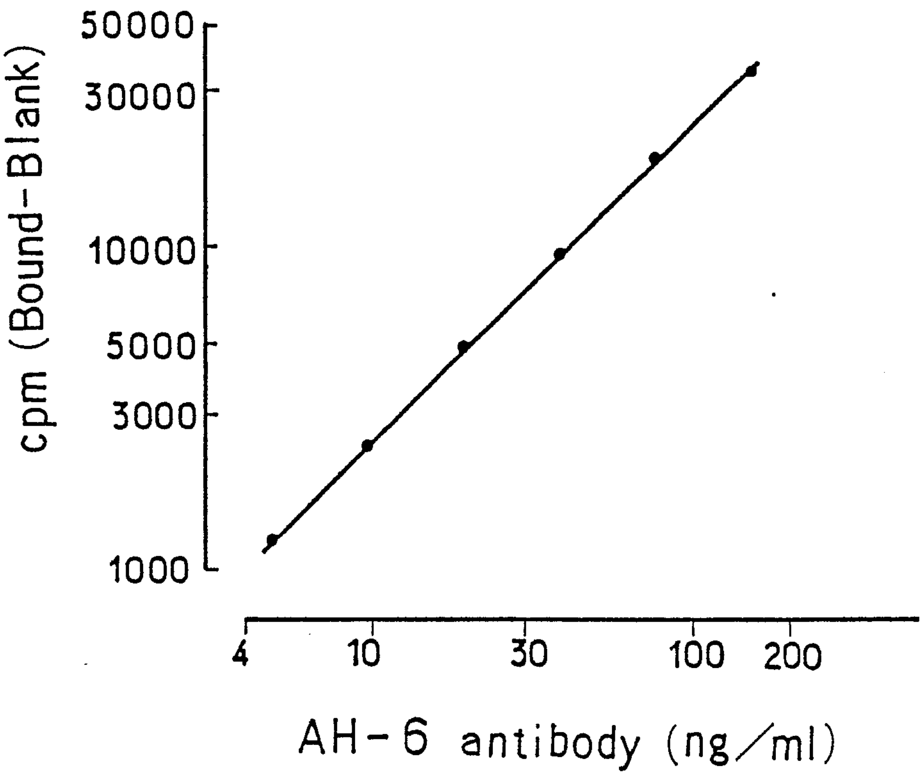

50000
30000
10000
5000
3000
1000
cpm (Bound-Blank)
4
10
30
100
200
AH-6 antibody (ng/ml)

FIG. 21

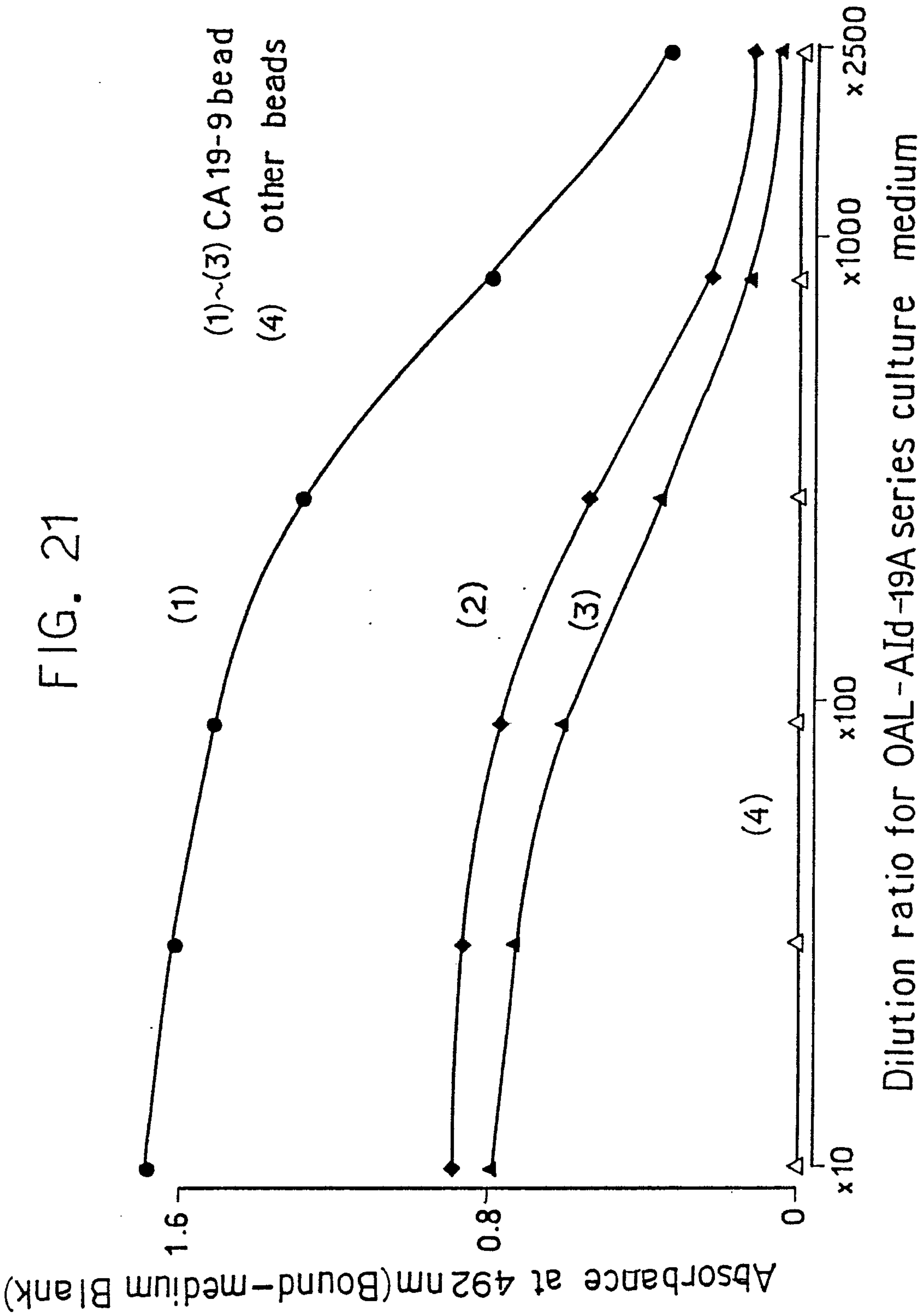
(1)~(3) CA19-9 bead
(4) other beads
(1)
(2)
(3)
(4)
1.6
0.8
0
x10
x100
x1000
x2500
Absorbance at 492 nm (Bound-medium Blank)
Dilution ratio for OAL-AId-19A series culture medium

FIG. 22

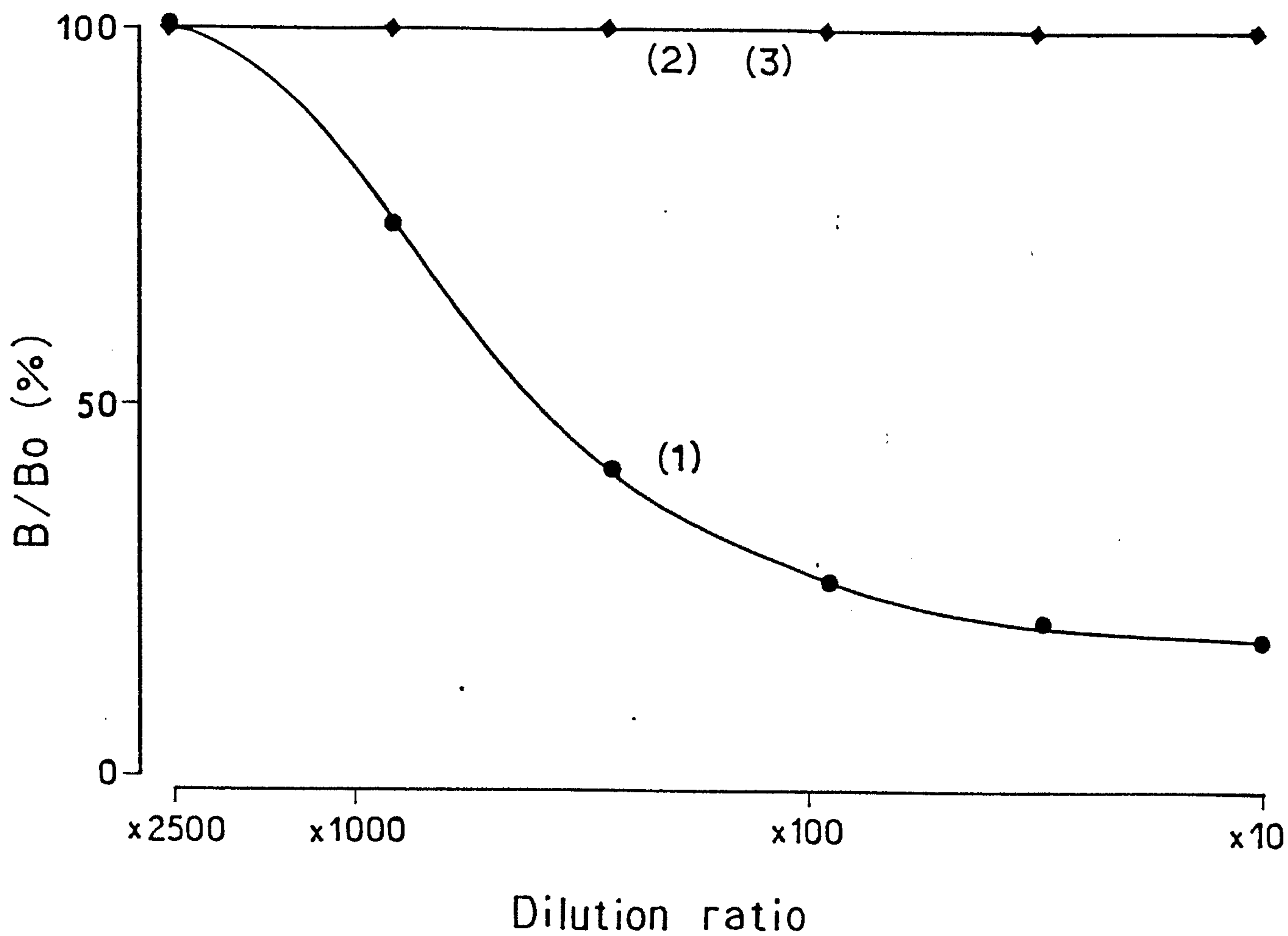

FIG. 23

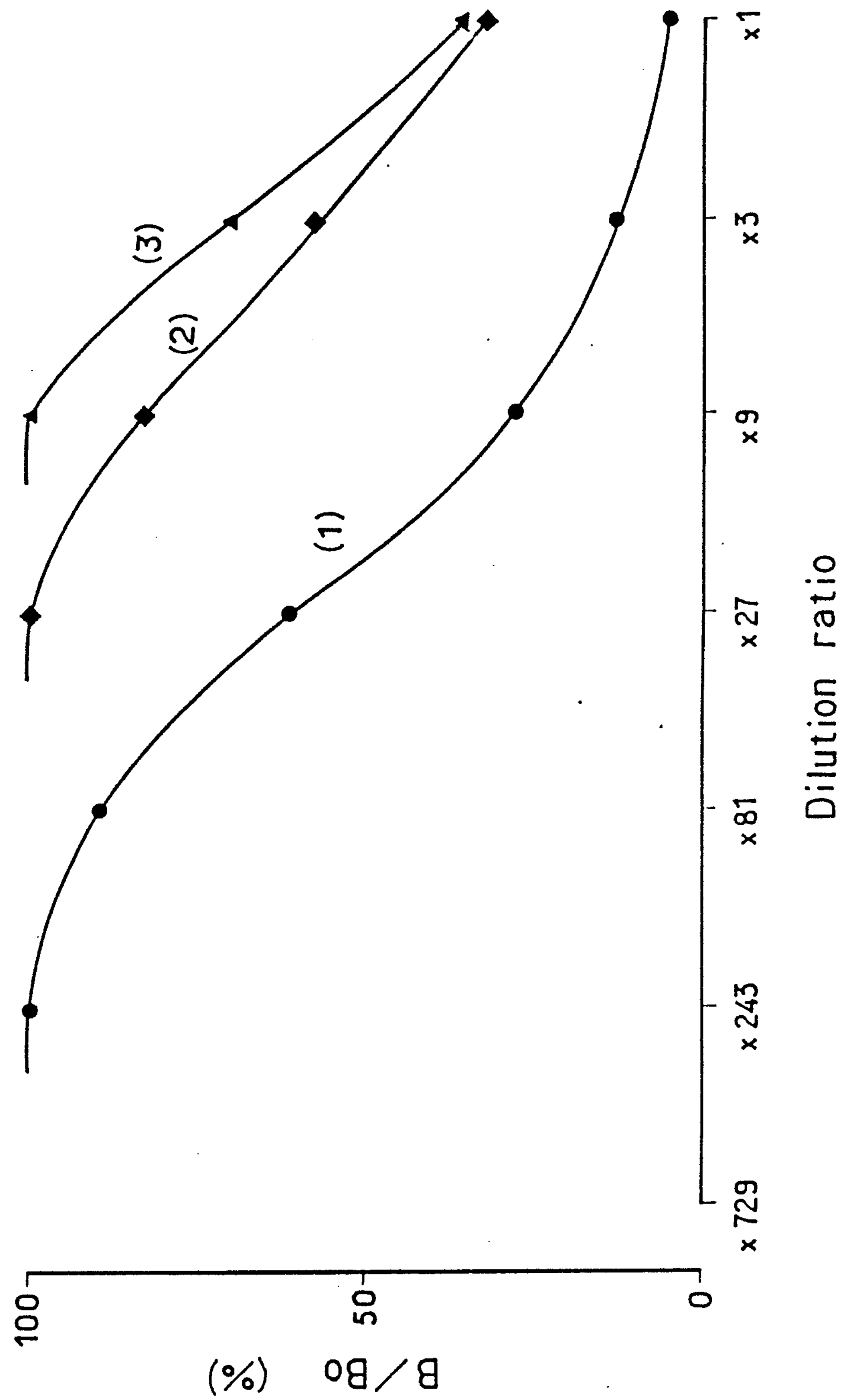

FIG. 24

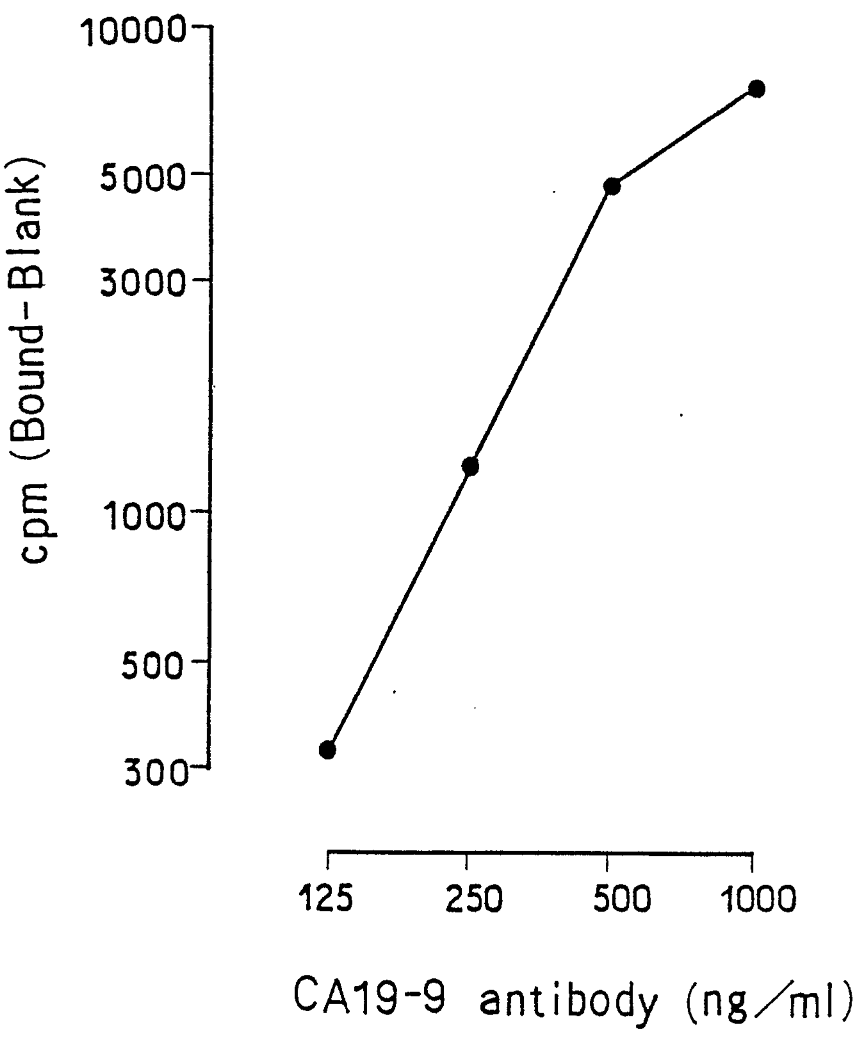
10000
5000
3000
1000
500
300
cpm (Bound-Blank)
125
250
500
1000
CA19-9 antibody (ng/ml)

FIG. 25

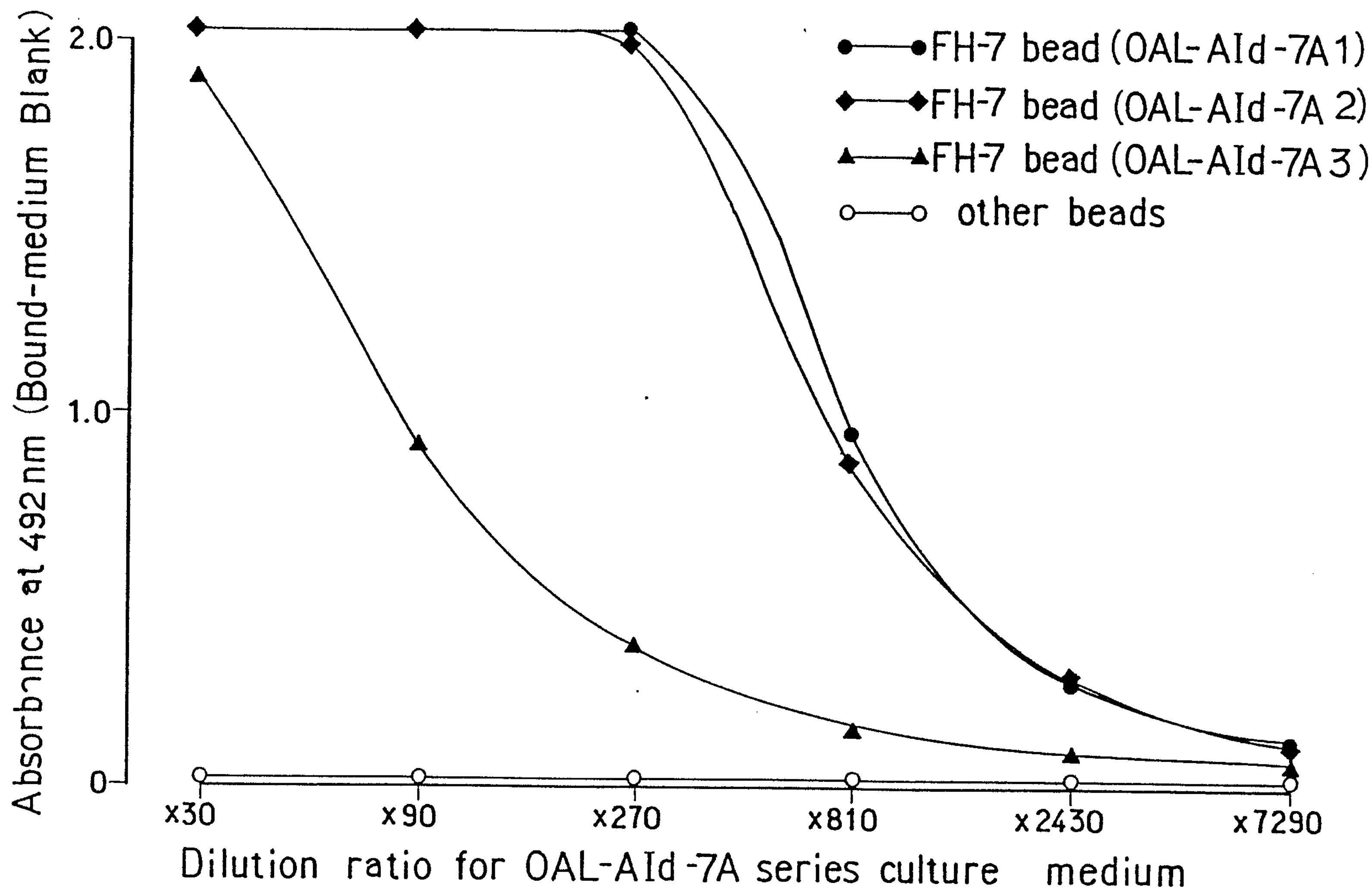
Absorbance at 492nm (Bound-medium Blank)
2.0
1.0
0
x30
x90
x270
x810
x2430
x7290
Dilution ratio for OAL-AId-7A series culture medium
FH-7 bead (OAL-AId-7A1)
FH-7 bead (OAL-AId-7A 2)
FH-7 bead (OAL-AId-7A 3)
other beads

FIG. 26

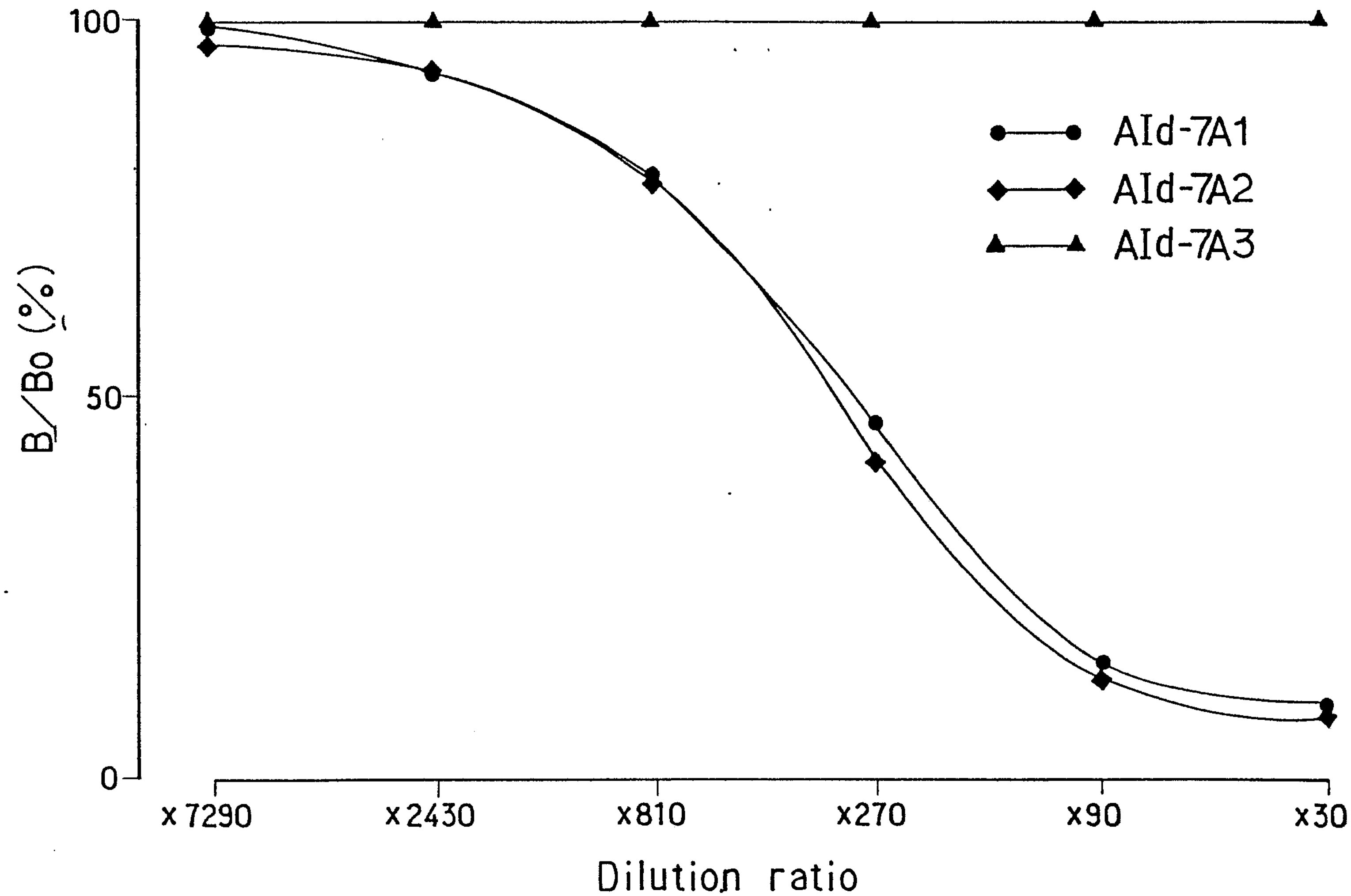
AId-7A1
AId-7A2
AId-7A3
100
50
0
B/Bo (%)
x 7290
x 2430
x810
x270
x90
x30
Dilution ratio

FIG. 27

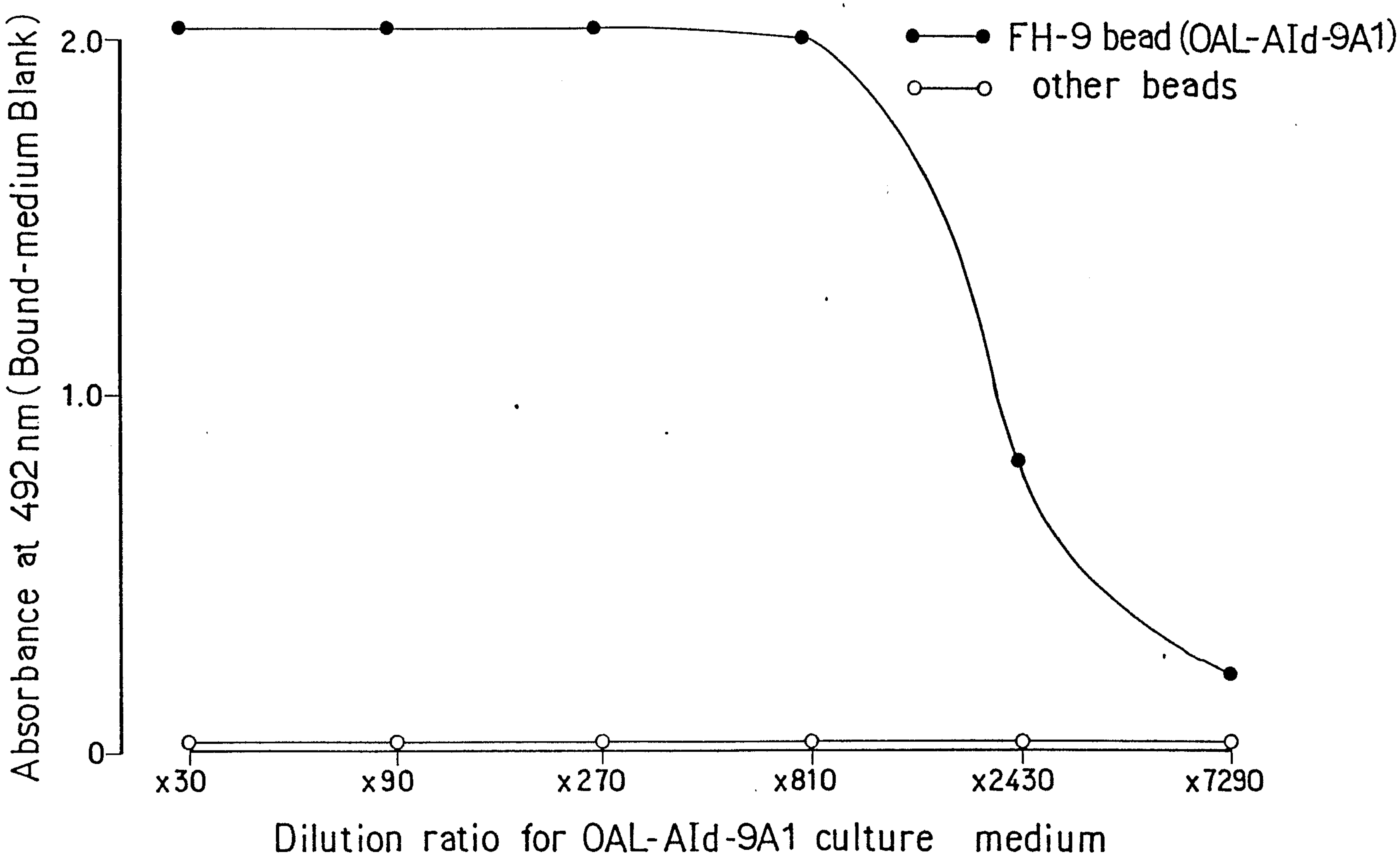
FH-9 bead (OAL-AId-9A1)
other beads
Absorbance at 492 nm ( Bound-medium Blank )
2.0
1.0
0
x30
x90
x270
x810
x2430
x7290
Dilution ratio for OAL-AId-9A1 culture medium

FIG. 28